# EUROPÄISCHE PATENTSCHRIFT

(11) **EP 2 170 925 B1**
(45) Veröffentlichungstag und Bekanntmachung des Hinweises auf die Patenterteilung: **24.10.2012**
(21) Anmeldenummer: 08758990.9
(22) Anmeldetag: 04.06.2008
(51) Int. Cl.: C07J 53/00, C07J 41/00, A61K 31/56, A61P 5/34

(54) **17ß-CYANO-19-NOR-ANDROST-4-EN-DERIVAT, DESSEN VERWENDUNG UND DAS DERIVAT ENTHALTENDE ARZNEIMITTEL**
17ß -CYANO-19-NOR-ANDROST-4-ENE DERIVATIVE, USE THEREOF AND MEDICAMENTS CONTAINING SAID DERIVATIVE
DÉRIVÉ DE 17ß -CYANO-19-NOR-ANDROST-4-ÈNE, SON UTILISATION, ET MÉDICAMENT CONTENANT CE DÉRIVÉ

(30) Priorität: 12.06.2007 DE 102007027637
(43) Veröffentlichungstag der Anmeldung: 07.04.2010
(73) Patentinhaber: Bayer Intellectual Property GmbH, 40789 Monheim (DE)
(72) Erfinder: KUHNKE, Joachim, 14482 Potsdam (DE); HUEBNER, Jan, 10317 Berlin (DE); BOHLMANN, Rolf, 14055 Berlin (DE); FRENZEL, Thomas, 65719 Hofheim (DE); KLAR, Ulrich, 13503 Berlin (DE); MENGES, Frederik, 69198 Schriesheim (DE); RING, SVEN, 07749 Jena (DE); BORDEN, Steffen, 13355 Berlin (DE); MUHN, Hans-Peter, 13465 Berlin (DE); PRELLE, Katja, 13465 Berlin (DE)
(74) Vertreter: Bayer Intellectual Property GmbH
(86) Internationale Anmeldenummer: PCT/EP2008/004429
(87) Internationale Veröffentlichungsnummer: WO 2008/151746

(56) Entgegenhaltungen:
- EP-A- 0 785 211
- WO-A-85/00609
- DE-A1- 2 226 552
- GB-A- 1 308 849
- US-A- 3 043 833
- US-A- 3 400 136
- US-A- 3 463 776
- US-A- 4 252 800
- WILKS J M ET AL: "Steroid binding specificity of the hamster uterine progesterone receptor" STEROIDS, ELSEVIER SCIENCE PUBLISHERS, NEW YORK, NY, US, Bd. 35, Nr. 6, 1. Juni 1980 (1980-06-01), Seiten 697-706, XP023430988 ISSN: 0039-128X [gefunden am 1980-06-01]
- DE GOOYER M E ET AL: "Receptor profiling and endocrine interactions of tibolone" STEROIDS, ELSEVIER SCIENCE PUBLISHERS, NEW YORK, NY, US, Bd. 68, Nr. 1, 1. Januar 2003 (2003-01-01), Seiten 21-30, XP004398468 ISSN: 0039-128X
- JEAN-CHRISTOPHE DORÉ ET AL: "Correspondance Analysis Applied to Steroid Receptor Binding" JOURNAL OF MEDICINAL CHEMISTRY, US AMERICAN CHEMICAL SOCIETY. WASHINGTON, Bd. 29, Nr. 1, 1. Januar 1986 (1986-01-01), Seiten 54-60, XP002018802 ISSN: 0022-2623
- SALMAN M ET AL: "A POTENTIAL RADIOIODINATED LIGAND FOR ANDROGEN RECEPTOR 7-ALPHA METHYL-17-ALPHA-2'-E-IODOVINYL-19-NORTESTO STERONE" JOURNAL OF MEDICINAL CHEMISTRY, US AMERICAN CHEMICAL SOCIETY. WASHINGTON, Bd. 34, Nr. 3, 1. Januar 1991 (1991-01-01), Seiten 1019-1024, XP002192593 ISSN: 0022-2623

## Beschreibung

Die Erfindung betrifft bestimmte 17β-Cyano-19-nor-androst-4-en-Derivate, deren Verwendung sowie die Derivate enthaltende Arzneimittel mit gestagener Wirkung, beispielsweise zur Behandlung von prä-, peri- und postmenopausalen sowie von prämenstruellen Beschwerden.

Aus der Literatur sind Verbindungen mit gestagener, antimineralcorticoider, antiandrogener oder antiestrogener Wirkung auf Basis eines Steroidgerüstes bekannt, welche beispielsweise von 19-Nor-androst-4-en-3-on oder einem Derivat davon abgeleitet sind (die Nummerierung des Steroidgerüstes ist beispielsweise Fresenius/Görlitzer 3.Aufl. 1991 "Organisch=chemische Nomenklatur" S. 60 ff. zu entnehmen).

So offenbart WO 2006072467 A1 die als Gestagen wirkende Verbindung 6β,7β-15β,16β-Dimethylen-3-oxo-17-pregn-4-en-21,17β-carbolacton (Drospirenon), welche beispielsweise in einem oralen Kontrazeptivum sowie einem Präparat zur Behandlung postmenopausaler Beschwerden verwendet wurde. Aufgrund seiner vergleichsweise geringen Affinität zum Gestagenrezeptor und seiner vergleichsweise hohen Ovulationshemmdosis ist Drospirenon in dem Kontrazeptivum jedoch in der relativ hohen täglichen Dosis von 3 mg enthalten. Drospirenon zeichnet sich darüber hinaus auch dadurch aus, dass es zusätzlich zur gestagenen Wirkung über aldosteronantagonistische (antimineralcorticoide) sowie antiandrogene Wirkung verfügt. Diese beiden Eigenschaften machen Drospirenon in seinem pharmakologischen Profil dem natürlichen Gestagen Progesteron sehr ähnlich, welches aber anders als Drospirenon nicht ausreichend oral bioverfügbar ist. Um die zu verabreichende Dosis zu senken, werden in WO 2006072467 A1 weiter ein 18-Methyl-19-nor-17-pregn-4-en-21,17-carbolacton sowie diese enthaltende pharmazeutische Präparate vorgeschlagen, welche über eine höhere gestagene Potenz als Drospirenon verfügen.

Daneben offenbart beispielsweise US-A 3,705,179 Steroide, welche eine antiandrogene Aktivität aufweisen und sich zur Behandlung von Krankheiten eignen, die im Zusammenhang mit Androgenen stehen.

In DE 22 26 552 B2 sind weiter 17-Cyano-19-nor-androst-4-en-3-on-Verbindungen beschrieben, welche progestomimetische, antiandrogene sowie antiestrogene Wirkungen mit exogenem Charakter zeigen.

Die Aufgabe der vorliegenden Erfindung ist es, Verbindungen zur Verfügung zu stellen, die über eine starke Bindung an den Gestagenrezeptor verfügen. Außerdem sollen die Verbindungen bevorzugt auch eine antimineralcorticoide Wirkung aufweisen.

Diese Aufgabe wird durch die erfindungsgemäßen 17β-Cyano-19-nor-androst-4-en-Derivate gemäß Anspruch 1, die Verwendung der erfindungsgemäßen Derivate gemäß Anspruch 2 sowie ein mindestens ein erfindungsgemäßes Derivat enthaltendes Arzneimittel gemäß Anspruch 4 gelöst. Vorteilhafte Ausführungsformen der Erfindung sind in den Unteransprüchen angegeben.

Die vorliegende Erfindung betrifft demnach ein 17β-Cyano-19-nor-androst-4-en-Derivat mit der allgemeinen chemischen Formel 1 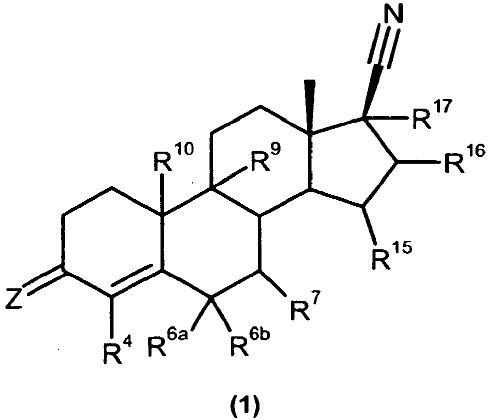 , namentlich die im Anspruch 1 spezifisch genannten Verbindungen.

Die Nummerierung des C-Gerüstes des erfindungsgemäßen Derivates mit der allgemeinen chemischen Formel **1** folgt in üblicher Weise der Nummerierung eines Steroid-Gerüstes, beispielsweise beschrieben in Fresenius, *a.a.O*. Die Nummerierung der in den Ansprüchen angegebenen Reste entspricht in analoger Weise ihrer Bindungsposition am C-Gerüst des Derivates. So bindet beispielsweise der Rest R⁴ an die C⁴-Position des erfindungsgemäßen Derivates.

Die erfindungsgemäßen Derivate können auch in Form von Solvaten, insbesondere von Hydraten vorliegen, wobei die erfindungsgemäßen Verbindungen demgemäß polare Lösungsmittel, insbesondere Wasser, als Strukturelement des Kristallgitters der erfindungsgemäßen Verbindungen enthalten. Das polare Lösungsmittel, insbesondere Wasser, kann in einem stöchiometrischen oder auch unstöchiometrischen Verhältnis vorliegen. Bei stöchiometrischen Solvaten, Hydraten spricht man auch von Hemi-, (Semi-), Mono-, Sesqui-, Di-, Tri-, Tetra-, Penta-, usw. Solvaten oder Hydraten.

Es wurde gefunden, dass die erfindungsgemäßen Verbindungen bzw. Derivate eine gute gestagene Wirkung *in vivo* aufweisen. Außerdem wirken einige interessante erfindungsgemäße Verbindungen als Antagonisten zum Mineralcorticoidrezeptor.

Die erfindungsgemäßen 17β-Cyano-19-nor-androst-4-en-Derivate, sind ausgewählt aus der Gruppe:

| | |
|---|---|
| 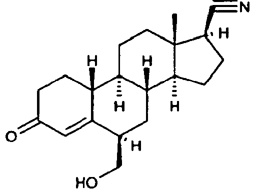 | 17β-Cyano-6β-hydroxymethylen-19-nor-androst-4-en-3-on |
| 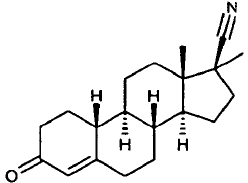 | 17β-Cyano-17α-methyl-19-nor-androst-4-en-3-on |
| 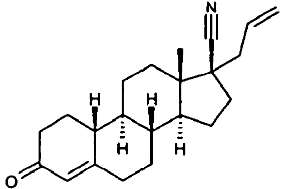 | 17α-Allyl-17β-cyano-19-nor-androst-4-en-3-on |
| 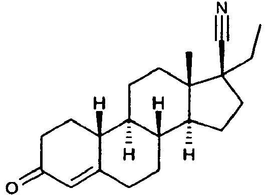 | 17β-Cyano-17α-ethyl-19-nor-androst-4-en-3-on |
| 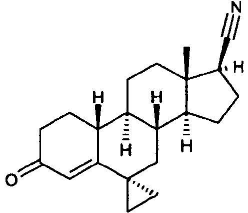 | 17β-Cyano-6,6-ethandiyl-19-nor-androst-4-en-3-on |
| 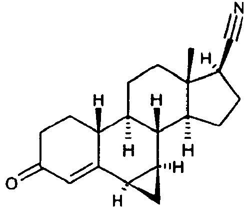 | 17β-Cyano-6β, 7β-methylen-19-nor-androst-4-en-3-on |
| 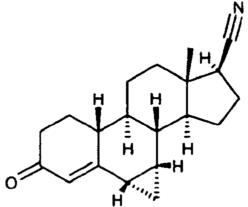 | 17β-Cyano-6α, 7α-methylen-19-nor-androst-4-en-3-on |
| 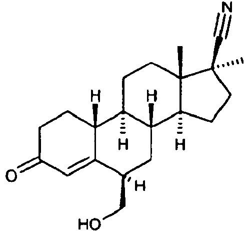 | 17β-Cyano-17α-methyl-6β-hydroxymethylen-19-nor-androst-4-en-3-on |
| 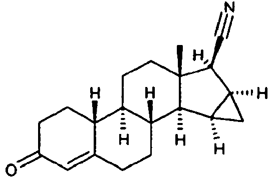 | 17β-Cyano-15β, 16β-methylen-19-nor-androst-4-en-3-on |
| 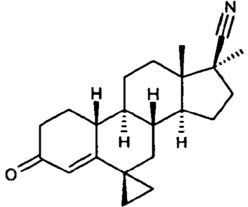 | 17β-Cyano-6,6-ethandiyl-17α-methyl-19-nor-androst-4-en-3-on |
| 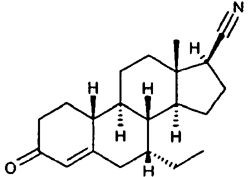 | 17β-Cyano-7α-ethyl-19-nor-androst-4-en-3-on |
| 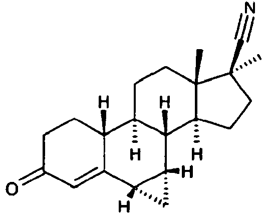 | 17β-Cyano-17α-methyl-6α, 7α-methylen-19-nor-androst-4-en-3-on |
| 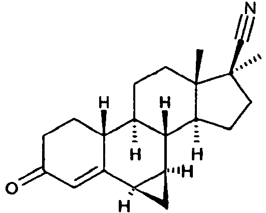 | 17β-Cyano-17α-methyl-6β, 7β-methylen-19-nor-androst-4-en-3-on |
| 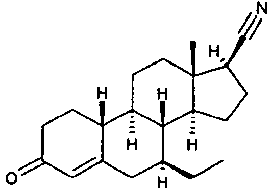 | 17β-Cyano-7β-ethyl-19-nor-androst-4-en-3-on |
| 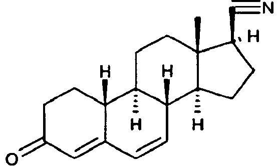 | 17β-Cyano-19-nor-androsta-4,6-dien-3-on |
| 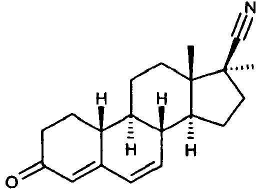 | 17β-Cyano-17α-methyl-19-nor-androsta-4,6-dien-3-on |
| 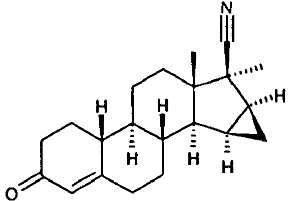 | 17β-Cyano-17α-methyl-15β, 16β-methylen-19-nor-androst-4-en-3-on |
| 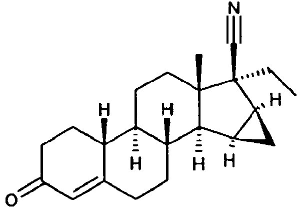 | 17β-Cyano-17α-ethyl-15β,16β-methylen-19-nor-androst-4-en-3-on |
| 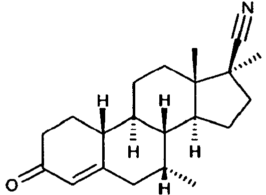 | 7α, 17α-Bismethyl-17β-cyano -19-nor-androst-4-en-3-on |
| 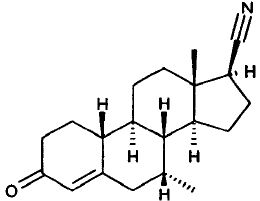 | 17β-Cyano-7α-methyl-19-nor-androst-4-en-3-on |
| 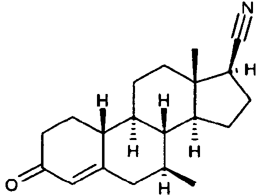 | 17β-Cyano-7β-methyl-19-nor-androst-4-en-3-on |
| 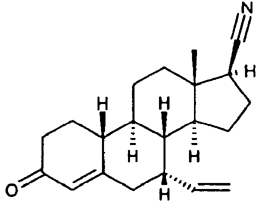 | 17β-Cyano-7α-vinyl-19-nor-androst-4-en-3-on |
| 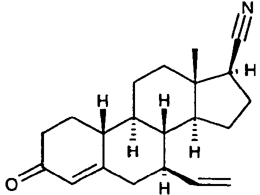 | 17β-Cyano-7β-vinyl-19-nor-androst-4-en-3-on |
| 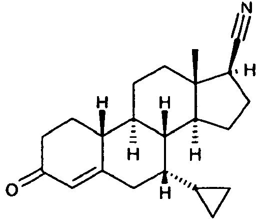 | 17β-Cyano-7α-cyclopropyl-19-nor-androst-4-en-3-on |
| 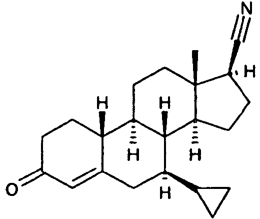 | 17β-Cyano-7β-cyclopropyl-19-nor-androst-4-en-3-on |
| 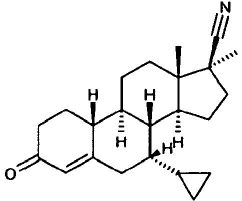 | 17β-Cyano-7α-cyclopropyl-17α-methyl-19-nor-androst-4-en-3-on |
| 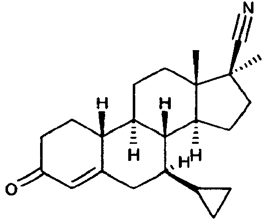 | 17β-Cyano-7β-cyclopropyl-17α-methyl-19-nor-androst-4-en-3-on |
| 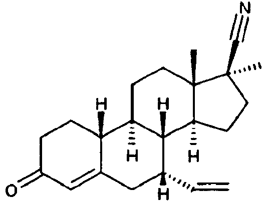 | 17β-Cyano-17α-methyl-7α-vinyl-19-nor-androst-4-en-3-on |
| 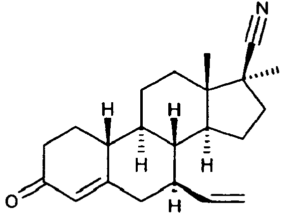 | 17β-Cyano-17α-methyl-7β-vinyl-19-nor-androst-4-en-3-on |
| 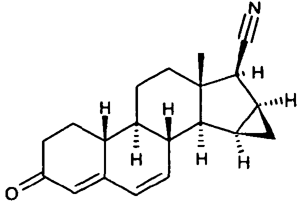 | 17β-Cyano-15β,16β-methylen-19-nor-androsta-4,6-dien-3-on |
| 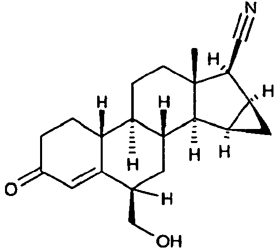 | 17β-Cyano-15β,16β-methylen-6β-hydroxymethyl-19-nor-androst-4-en-3-on |
| 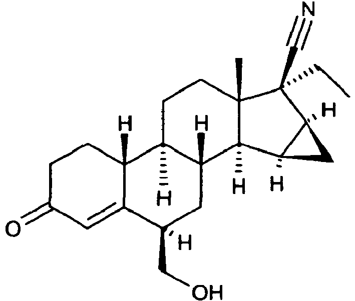 | 17α -Ethyl-17β-cyano-15β,16β-methylen-6β-hydroxymethyl-19-nor-androst-4-en-3-on |
| 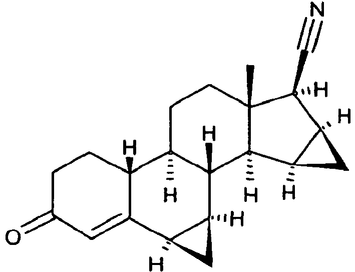 | 17β-Cyano-6β,7β-15β,16β-bismethylen-19-nor-androst-4-en-3-on |
| 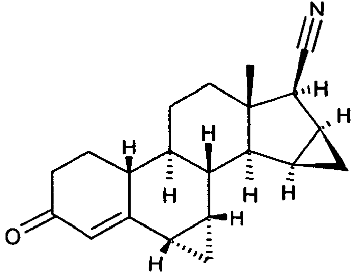 | 17β-Cyano-6α,7α-15β,16β-bismethylen-19-nor-androst-4-en-3-on |
| 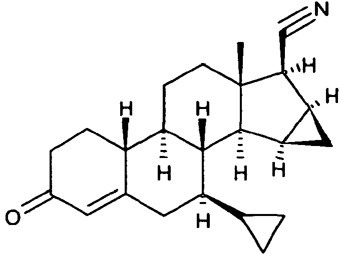 | 17β-Cyano-7β-cyclopropyl-15β,16β-methylen-19-nor-androst-4-en-3-on |
| 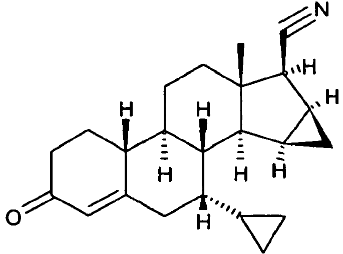 | 17β-Cyano-7α-cyclopropyl-15β,16β-methylen-19-nor-androst-4-en-3-on |
| 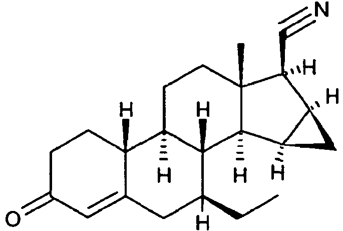 | 17β-Cyano-7β-ethyl-15β,16β-methylen-19-nor-and rost-4-en-3-on |
| 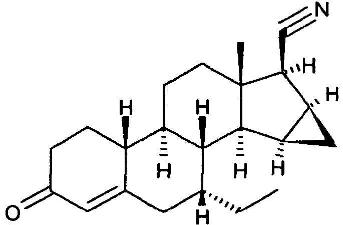 | 17β-Cyano-7a-ethyl-15β,16β-methylen-19-nor-androst-4-en-3-on |
| 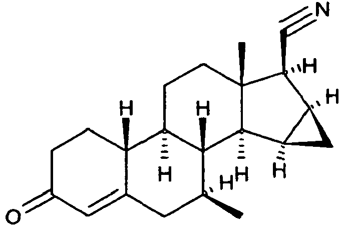 | 17β-Cyano-7β-methyl-15β,16β-methylen-19-nor-androst-4-en-3-on |
| 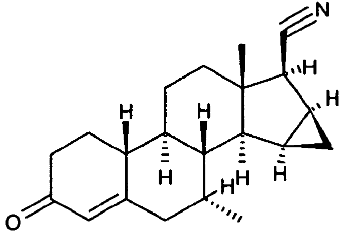 | 17β-Cyano-7α-methyl-15β,16β-methylen-19-nor-androst-4-en-3-on |
| 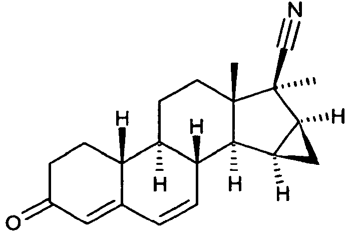 | 17β-Cyano-17α-methyl-15β,16β-methylen-19-nor-androsta-4,6-dien-3-on |
| 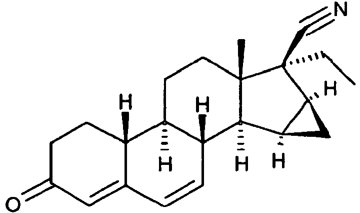 | 17β-Cyano-17α-ethyl-15β,16β-methylen-19-nor-androsta-4,6-dien-3-on |
| 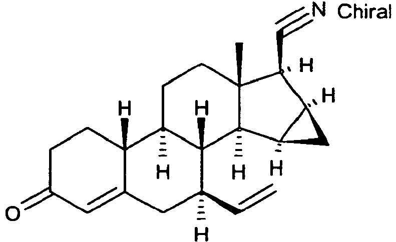 | 17β-Cyano-15β,16β-methylen-7β-vinyl-19-nor-androst-4-en-3-on |
| 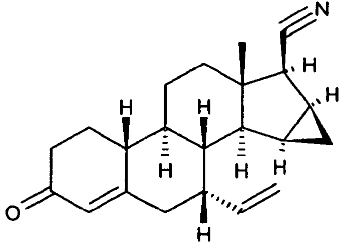 | 17β-Cyano-15β,16β-methylen-7α-vinyl-19-nor-androst-4-en-3-on |
| 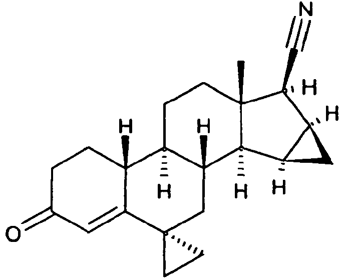 | 17β-Cyano-6,6-ethandiyl-15β,16β-methylen-19-nor-androst-4-en-3-on |
| 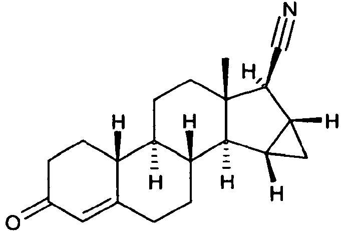 | 17β-Cyano-15α, 16α-methylen-19-nor-androst-4-en-3-on |
| 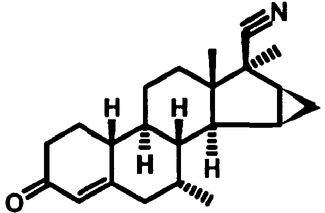 | 17β-Cyano-17α,7α-dimethyl-15β,16β-methylen-19-nor-androst-4-en-3-on |
| 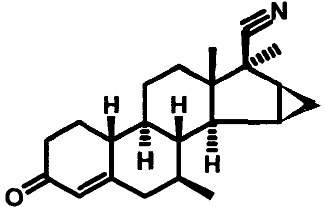 | 17β-Cyano-17α,7β-dimethyl-15β,16β-methylen-19-nor-androst-4-en-3-on |
| 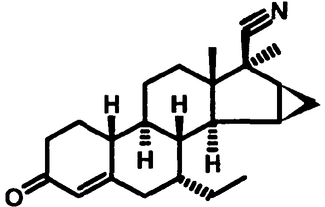 | 17β-Cyano-17α-methyl -7α-ethyl-15β,16β-methylen-19-nor-androst-4-en-3-on |
| 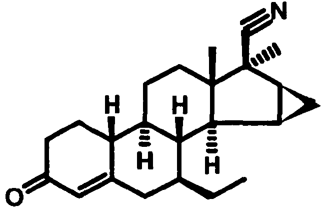 | 17β-Cyano-17a-methyl 7β-ethyl-15β,16β-methylen-19-nor-androst-4-en-3-on |
| 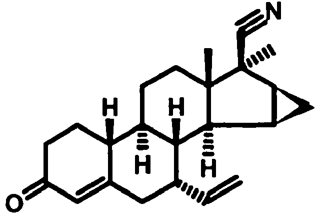 | 17β-Cyano-17α-methyl -7α-vinyl-15β,16β-methylen-19-nor-androst-4-en-3-on |
| 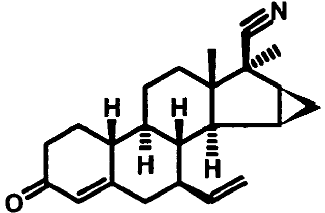 | 17β-Cyano-17α-methyl -7β-vinyl-15β,16β-methylen-19-nor-androst-4-en-3-on |
| 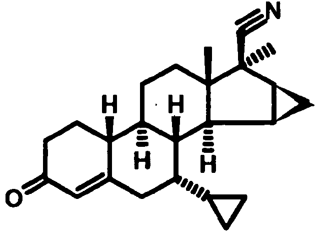 | 17β-Cyano-17α-methyl-7α-cyclopropyl-15β,16β-methylen-19-nor-androst-4-en-3-on |
| 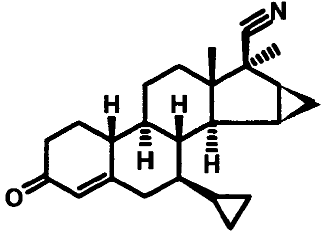 | 17β-Cyano-17α-methyl-7β-cyclopropyl-15β,16β-methylen-19-nor-androst-4-en-3-on |
| 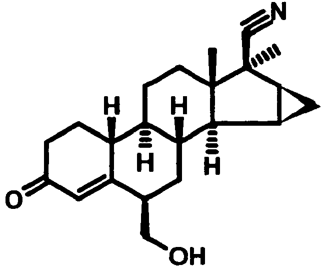 | 17β-Cyano-17α-methyl-6β-hydroxymethyl-15β,16β-methylen-19-nor-androst-4-en-3-on |
| 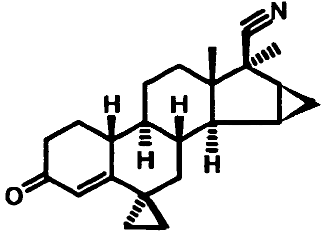 | 17β-Cyano-17α-methyl-6,6-ethylen-15β,16β-methylen-19-nor-androst-4-en-3-on |
| 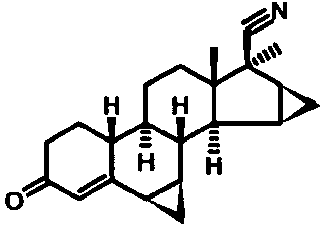 | 17β-Cyano-17α-methyl-6β,7β-methylen-15β,16β-methylen-19-nor-androst-4-en-3-on |
| 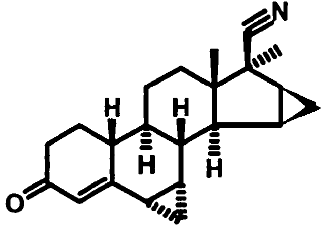 | 17β-Cyano-17α-methyl-6α,7α-methylen-15β,16β-methylen-19-nor-androst-4-en-3-on |
| 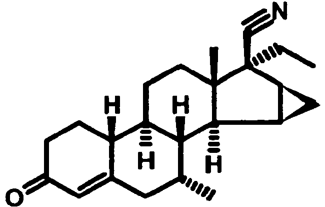 | 17β-Cyano-17α-ethyl-7α-methyl-15β,16β-methylen-19-nor-androst-4-en-3-on |
| 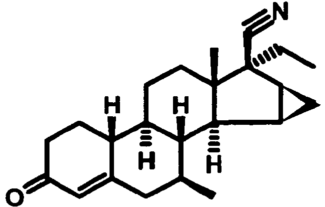 | 17β-Cyano-17a-ethyl-7β-methyl 15β,16β-methylen-19-nor-androst-4-en-3-on |
| 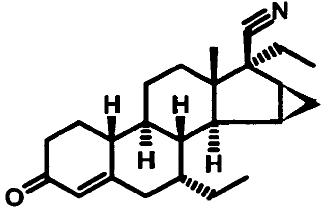 | 17β-Cyano-17α,7α-diethyl-15β,16β-methylen-19-nor-androst-4-en-3-on |
| 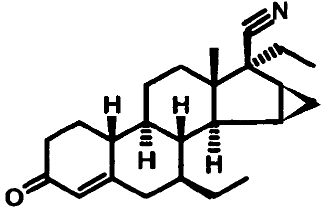 | 17β-Cyano-17α,7β-diethyl-15β,16β-methylen-19-nor-androst-4-en-3-on |
| 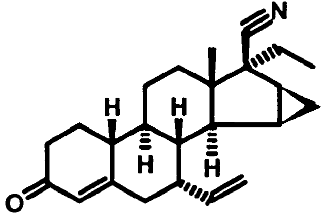 | 17β-Cyano-17a-ethyl -7α-vinyl-15β,16β-methylen-19-nor-androst-4-en-3-on |
| 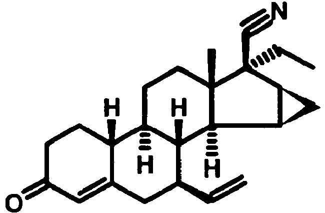 | 17β-Cyano-17α-ethyl-7β-vinyl-15β,16β-methylen-19-nor-androst-4-en-3-on |
| 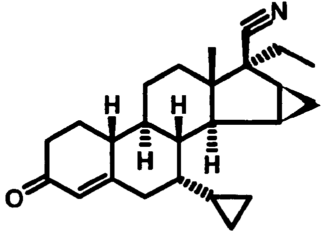 | 17β-Cyano-17α-ethyl-7α-cyclopropyl-15β,16β-methylen-19-nor-androst-4-en-3-on |
| 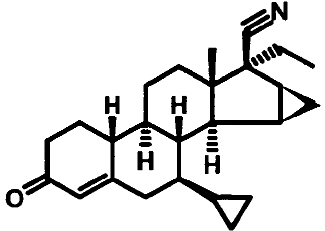 | 17β-Cyano-17α-ethyl-7β-cyclopropyl-15β,16β-methylen-19-nor-androst-4-en-3-on |
| 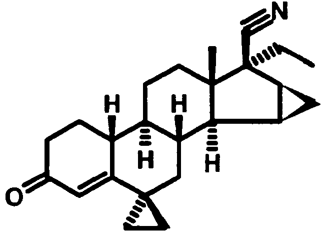 | 17β-Cyano-17α-ethyl-6,6-ethylen-15β,16β-methylen-19-nor-androst-4-en-3-on |
| 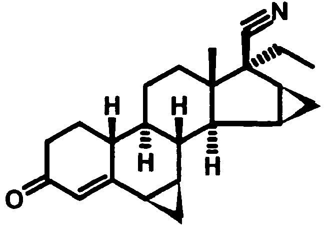 | 17β-Cyano-17α-ethyl-6β,7β-methylen-15β,16β-methylen-19-nor-androst-4-en-3-on |
| 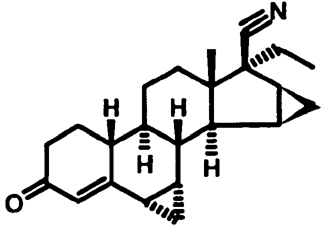 | 17β-Cyano-17α-ethyl-6α,7α-methylen-15β,16β-methylen-19-nor-androst-4-en-3-on |

Ganz besonders bevorzugt sind die 15α,16α- und die 15β,16β-Methylen-Derivate in der vorstehenden Liste.

Aufgrund ihrer gestagenen Wirksamkeit können die neuen obengenannten erfindungsgemäßen Verbindungen allein oder in Kombination mit Estrogenen in Arzneimitteln zur Kontrazeption verwendet werden.

Die erfindungsgemäßen Derivate eignen sich daher insbesondere zur Herstellung eines Arzneimittels zur oralen Kontrazeption und zur Behandlung von prä-, peri- und postmenopausalen Beschwerden, einschließlich der Verwendung in Präparaten für die Hormon-Substitutionstherapie (HRT).

Wegen ihres günstigen Wirkungsprofils sind die erfindungsgemäßen Derivate außerdem besonders gut geeignet zur Behandlung prämenstrueller Beschwerden, wie Kopfschmerzen, depressiver Verstimmungen, Wasserretention und Mastodynie.

Besonders bevorzugt ist die Verwendung der erfindungsgemäßen Derivate zur Herstellung eines Arzneimittels mit gestagener und antimineralcorticoider Wirkung.

Eine Behandlung mit den erfindungsgemäßen Derivaten findet bevorzugt am Menschen statt, kann aber auch an verwandten Säugetierspezies, wie beispielsweise an Hund und Katze, durchgeführt werden.

Zur Verwendung der erfindungsgemäßen Derivate als Arzneimittel werden diese mit mindestens einem geeigneten pharmazeutisch unbedenklichen Zusatzstoff, beispielsweise Trägerstoff, kombiniert. Der Zusatzstoff ist beispielsweise für die parenterale, vorzugsweise orale, Applikation geeignet. Es handelt sich dabei um pharmazeutisch geeignete organische oder anorganische inerte Zusatzmaterialien, wie zum Beispiel, Wasser, Gelatine, Gummi arabicum, Milchzucker, Stärke, Magnesiumstearat, Talk, pflanzliche Öle, Polyalkylenglykole usw. Die Arzneimittel können in fester Form, zum Beispiel als Tabletten, Dragees, Suppositorien, Kapseln, oder in flüssiger Form, zum Beispiel als Lösungen, Suspensionen oder Emulsionen, vorliegen. Gegebenenfalls enthalten sie darüber hinaus Hilfsstoffe, wie Konservierungs-, Stabilisierungs-, Netzmittel oder Emulgatoren, Salze zur Veränderung des osmotischen Druckes oder Puffer. Für die parenterale Applikation sind insbesondere ölige Lösungen, wie zum Beispiel Lösungen in Sesamöl, Rizinusöl und Baumwollsamenöl, geeignet. Zur Erhöhung der Löslichkeit können Lösungsvermittler, wie zum Beispiel Benzylbenzoat oder Benzylalkohol, zugesetzt werden. Es ist auch möglich, die erfindungsgemäßen Derivate in ein transdermales System einzuarbeiten und sie damit transdermal zu applizieren. Für die orale Applikation kommen insbesondere Tabletten, Dragees, Kapseln, Pillen, Suspensionen oder Lösungen in Frage.

Die Dosierung der erfindungsgemäßen Derivate in Kontrazeptionspräparaten soll 0,01 bis 10 mg pro Tag betragen. Die Tagesdosis bei der Behandlung prämenstrueller Beschwerden liegt bei etwa 0,1 bis 20 mg. Die erfindungsgemäßen gestagenen Derivate werden in Kontrazeptionspräparaten sowie in den Arzneimitteln zur Behandlung prämenstrueller Beschwerden vorzugsweise oral appliziert. Die tägliche Dosis wird vorzugsweise einmalig verabreicht.

Die gestagenen und estrogenen Wirkstoffkomponenten werden in Kontrazeptionspräparaten vorzugsweise zusammen oral appliziert. Die tägliche Dosis wird vorzugsweise einmalig verabreicht.

Als Estrogene kömmen synthetische Estrogene, vorzugsweise Ethinylestradiol, aber auch Mestranol in Betracht.

Das Estrogen wird in einer täglichen Menge verabfolgt, die der von 0,01 bis 0,04 mg Ethinylestradiol entspricht.

Als Estrogene in den Arzneimitteln zur Behandlung von prä-, peri- und postmenopausalen Beschwerden sowie für die Hormon-Substitutionstherapie kommen in erster Linie natürliche Estrogene zur Anwendung, vor allem das Estradiol oder dessen Ester, beispielsweise Estradiolvalerat, oder auch konjugierte Estrogene (CEEs = Conjugated Equine Estrogens).

Soweit die Herstellung der Ausgangsverbindungen hier nicht beschrieben ist, sind diese dem Fachmann bekannt oder analog zu bekannten Verbindungen oder hier beschriebenen Verfahren herstellbar. Die Isomerengemische können nach üblichen Methoden, wie beispielsweise Kristallisation, Chromatographie oder Salzbildung, in die Enantiomeren, E/Z-Isomeren bzw. Epimeren aufgetrennt werden..

Die erfindungsgemäßen Derivate werden wie nachstehend beschrieben hergestellt.

Als Startmaterialien für die hier beschriebenen 17β-Cyano-19-nor androst-4-en-3-on-Derivate eignen sich diverse steroidale Ausgangsmaterialien, wie beispielsweise 19-Nor-androst-4-en-3,17-dion, oder auch die teilreduzierten Analoga.

Mikrobiologisch ist beispielsweise 15-Hydroxy-19-nor-androst-4-en-3,17-dion zugänglich, welches einen Zugang zu 15β,16β-methylenierten 17-Cyanosteroiden eröffnet, siehe hierzu Beispiele im experimentellen Teil. 15α,16α-methylenierte Vorstufen, die für die Synthese der entsprechenden 17-Cyanosteroide geeignet sind, sind ebenfalls bekannt, z.B. 17β-hydroxy-15α,16α-methylen-19-nor-androst-4-en-3-on in DE-A 22 07 421 (1973). Ein Zugang zu 17β-Cyano-19-nor-androst-4-en-3-on ist in DE-A 22 26 552 beschrieben.

Dem Fachmann selbstverständlich ist, dass bei den Beschreibungen der synthetischen Transformationen immer vorausgesetzt wird, dass gegebenenfalls am Steroidgerüst vorhandene sonstige funktionelle Gruppen in geeigneter Form geschützt sind.

Die Einführung eines Nitrils in Position 17 (C¹⁷) des Steroidgerüstes kann auf vielfältige Weise erfolgen. Hier kommen sowohl einstufige Verfahren als auch mehrstufige Varianten in Betracht. Bevorzugt sind hier Methoden, die letztlich den Austausch einer Sauerstofffunktion gegen Cyanid bedeuten. Viele in Betracht kommende Verfahrensvarianten sind beschrieben in Science of Synthesis Houben-Weyl Methods of Molecular Transformations Category 3 Volume 19 S. 197-213 (2004 Georg Thieme Verlag Stuttgart, New York) sowie in Houben-Weyl Methoden der organischen Chemie Band E5 Teil 2 S. 1318-1527 (1985 Georg Thieme Verlag Stuttgart, New York).

Als Einstufenverfahren bietet sich beispielsweise der direkte reduktive Austausch eines Carbonylsauerstoffatoms gegen eine Cyanogruppe an. Hierzu wird ein 17-Ketosteroid mit Tosylmethylisocyanid in geeigneten Lösemitteln, wie etwa Dimethoxyethan, Dimethylsulfoxid, Ethern, Alkoholen oder auch deren Gemischen, unter Verwendung geeigneter Basen, wie etwa Alkalialkoholaten, Alkalihydriden, Kaliumhexamethyldisilazid, oder auch Alkaliamiden, wie etwa Lithiumdiisopropylamid, in einem Temperaturbereich von 0°C bis 100°C umgesetzt. Gegebenenfalls entstehende 17-Epimerengemische lassen sich durch Chromatographie, fraktionierte Kristallisation oder mit einer Kombination dieser Methoden trennen.

Auch der SN₂-artige Austausch einer geeigneten Abgangsgruppe an Position 17, wie etwa eines Halogenids (bevorzugt Jod oder Brom) oder auch eines Sulfonsäureesters eines 17-Alkoholes, gegen Cyanid kommt in Betracht. Als Cyanidquellen werden bevorzugt anorganische Cyanide, wie Lithium-, Natrium- und Kaliumcyanid, verwendet.

Als Beispiele für mehrstufige Varianten der Nitrileinführung seien die Folgenden genannt: Ein 17-Keton wird mittels einer Wittig-Olefinierung in die entsprechende 17-Exomethylenverbindung überführt, welche nach Hydroborierung und Oxidation zum Aldehyd zum entsprechenden 17-Carbaldehydoxim umgesetzt werden kann. Dehydratisierung des Oxims führt dann zum 17-Nitril.

Die Einführung des Nitriles kann sowohl am Anfang einer Synthesesequenz als auch zu einem beliebigen späteren Zeitpunkt durchgeführt werden, vorausgesetzt, dass gegebenenfalls vorhandene weitere funktionelle Gruppen in geeigneter Weise geschützt sind.

Die 17-Cyanoverbindungen lassen sich gegebenenfalls alkylieren, was zu stereochemisch einheitlichen 17β-Cyano-17α-substituierten Derivaten führt. Hierzu wird das 17-Cyanosteroid in einem geeigneten Lösemittel, wie etwa Ethern, beispielsweise Tetrahydrofuran, deprotoniert. Hier können diverse Basen verwendet werden, beispielsweise ein Alkaliamid, wie Lithiumdiisopropylamid. Nach Zusatz eines Alkylierungsmittels, wie etwa eines Alkyl- oder Alkenylhalogenides, und Aufarbeitung werden dann die 17β-Cyano-17α-substituierten Derivate erhalten.

Exemplarisch sei das weitere synthetische Vorgehen an Hand des folgenden Syntheseschemas erläutert, wobei als Edukt die bereits beschriebene Verbindung **2** (DE-A 22 26 552 (1972)) aufgeführt wird:

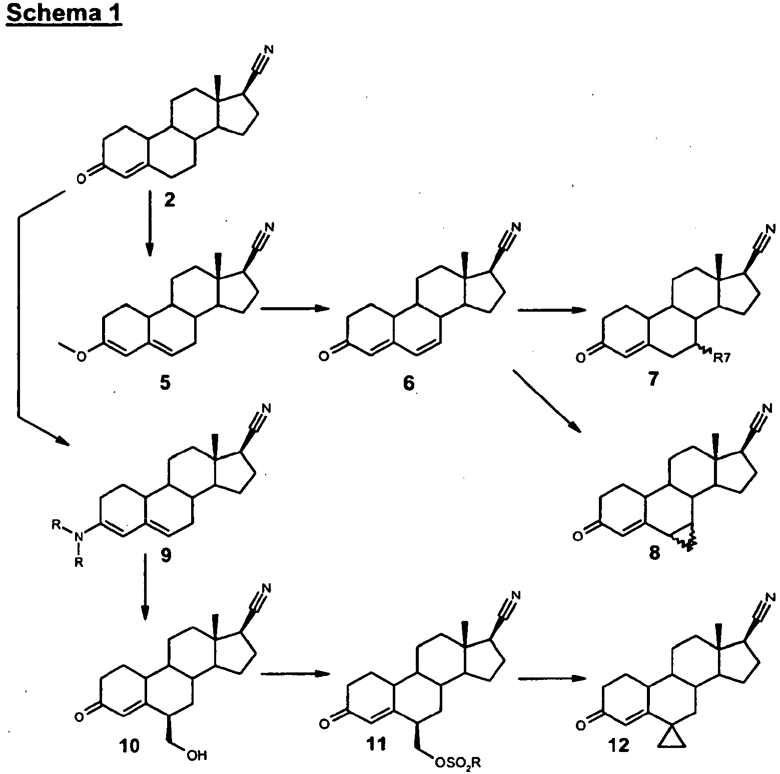

Die Einführung einer 6,7-Doppelbindung erfolgt über Bromierung des 3,5-Dienolethers **5** sowie anschließende Bromwasserstoffabspaltung (siehe z.B. J. Fried, J.A. Edwards, Organic Reactions in Steroid Chemistry, von Nostrand Reinhold Company 1972, S. 265-374).

Die Dienoletherbromierung von Verbindung **5** kann z.B. analog der Vorschrift aus Steroids 1, 233 (1963**)** erfolgen. Die Bromwasserstoffabspaltung gelingt durch Erhitzen der 6-Bromverbindung mit basischen Reagenzien, wie z.B. LiBr oder Li₂CO₃, in aprotischen Lösungsmitteln, wie Dimethylformamid, bei Temperaturen von 50°C bis 120°C oder aber indem die 6-Bromverbindungen in einem Lösungsmittel, wie Collidin oder Lutidin, erhitzt werden zu Verbindung **6.**

Verbindung **7** wird durch Methenylierung der 6,7-Doppelbindung nach bekannten Verfahren, z.B. mit Dimethylsulfoxoniummethylid (siehe z.B. DE-A 11 83 500, DE-A 29 22 500, EP-A 0 019 690, US-A 4,291,029; *J.* Am. Chem. Soc. 84, 867 (1962)) in eine Verbindung **8** umgewandelt, wobei ein Gemisch der α- und β-Isomeren erhalten wird, das z.B. durch Chromatographie in die einzelnen Isomeren getrennt werden kann.

Verbindungen vom Typ **7** können, wie in den Beispielen beschrieben, oder analog zu diesen Vorschriften unter Verwendung analoger zu den dort beschriebenen Reagenzien erhalten werden.

Die Synthese der spirocyclischen Verbindung **12** geht von **2** aus, welches zunächst in ein 3-Amino-3,5-dien-Derivat **9** überführt wird. Durch Umsetzung mit Formalin in alkoholischer Lösung wird das 6-Hydroxymethylen-Derivat **10** erhalten. Nach Überführung der Hydroxygruppe in eine Fluchtgruppe, wie etwa ein Mesylat, Tosylat (Verbindung **11)** oder auch Benzoat, lässt sich Verbindung **13** durch Umsetzung mit Trimethylsulfoxoniumiodid unter Verwendung von Basen, wie etwa Alkalihydroxiden, Alkalialkoholaten, in geeigneten Lösemitteln, wie etwa Dimethylsulfoxid, darstellen.

Die nachfolgenden Beispiele dienen der näheren Erläuterung der Erfindung:
Die erfindungsgemäßen Verbindungen zeichnen sich überraschenderweise durch starke gestagene Wirksamkeit aus und sind im Schwangerschaftserhaltungs-Test an der Ratte nach subcutaner Applikation stark wirksam.

Durchführung des Schwangerschaftserhaltungstests an der Ratte:
In graviden Ratten induziert die Entfernung der Corpora lutea oder Kastration einen Abort. Durch die exogene Zufuhr von Progestinen (Gestagenen) in Kombination mit einer geeigneten Dosis eines Estrogens gelingt die Aufrechterhaltung der Schwangerschaft. Der Schwangerschaftserhaltungstest an ovarektomierten Ratten dient zur Bestimmung der peripheren gestagenen Aktivität einer Verbindung.

Ratten wurden während des Proestrus über Nacht angepaart. Die Anpaarung wurde am Morgen des darauf folgenden Tages durch die Begutachtung eines Vaginalabstriches kontrolliert. Die Anwesenheit von Spermien wurde dabei als Tag 1 einer beginnenden Schwangerschaft bewertet. Am Tag 8 der Schwangerschaft wurden die Tiere unter Etheranesthesie ovarektomiert. Die Behandlung mit Testverbindung und exogenem Estrogen (Estron, 5 µg/kg/Tag) wurde von Tag 8 bis Tag 15 oder Tag 21 der Schwangerschaft einmal täglich subkutan ausgeführt. Die erste Anwendung am Tag 8 wurde zwei Stunden vor der Kastration ausgeführt. Intakte Kontrolltiere erhielten ausschließlich Vehikel.

### Auswertung:

Am Ende des Versuches (Tag 15 oder Tag 21) wurden die Tiere unter CO₂-Atmosphäre getötet, und es wurden lebende Föten (Föten mit schlagendem Herz) und Implantationsstellen (frühe Resorptionen und tote Föten einschließlich Autolyse und atrophische Plazenten) in beiden uterinen Hörnern gezählt. Am Tag 22 konnten Föten außerdem auf Missbildungen untersucht werden. In Uteri ohne Föten oder Implantationsstellen wurde die Anzahl von Nidationsstellen durch Anfärben mit 10%iger Ammoniumsulfid-Lösung ermittelt. Die Schwangerschaftserhaltungsrate wurde als Quotient aus der Anzahl der lebenden Föten und der gesamten Anzahl von Nidationsstellen (sowohl resorbierte und tote Föten als auch Nidationsstellen) berechnet. Für bestimmte Testsubstanzen wurden die in Tabelle 1 angegebenen die Schwangerschaft erhaltenden Dosen (ED50) ermittelt. Für Drospirenon beträgt dieser Wert 3,5 mg/kg/Tag.

Die erfindungsgemäßen Derivate mit der allgemeinen chemischen Formel **1** verfügen über eine sehr starke gestagene Wirksamkeit. Es wurde außerdem gefunden, dass die erfindungsgemäßen Derivate *in vitro* antimineralcorticoide Wirkung zeigen. Sie sollten deshalb *in vivo* kaliumretinierende, natriuretische (antimeralcorticoide) Wirkung besitzen. Diese Eigenschaften wurden mit dem nachfolgend beschriebenen Test ermittelt:
Zur Kultivierung der für den Assay verwendeten Zellen wurde als Kultivierungsmedium DMEM (Dulbecco's Modified Eagle Medium: 4500 mg/ml Glukose; PAA, #E15-009) mit 10% FCS (Biochrom, S0115, Charge #615B), 4 mM L-Glutamin, 1 % Penicillin/Streptomycin, 1 mg/ml G418 und 0,5 µg/ml Puromycin verwendet.

Reporter-Zelllinien wurden in einer Dichte von 4 x 10⁴ Zellen pro Vertiefung in weißen, undurchsichtigen Gewebekulturplatten mit jeweils 96 Vertiefungen angezüchtet (PerkinElmer, #P12-106-017) und in 6 % DCC-FCS (Aktivkohle behandeltes Serum, zur Entfernung im Serum enthaltener störender Komponenten) gehalten. Die zu untersuchenden Verbindungen wurden acht Stunden später zugegeben, und die Zellen wurden mit den Verbindungen 16 Stunden lang inkubiert. Die Versuche wurden dreifach ausgeführt. Am Ende der Inkubation wurde das Effektor enthaltende Medium entfernt und durch Lysis-Puffer ersetzt. Nachdem Luciferase-Assay-Substrat (Promega, #E1501) zugegeben worden war, wurden die Platten mit den 96 Vertiefungen dann in ein Mikroplatten-Luminometer (Pherastar, BMG labtech) eingeführt, und die Lumineszenz wurde gemessen. Die IC50-Werte wurden unter Verwendung einer Software zur Berechnung von Dosis-Wirkungsbeziehungen ausgewertet. In Tabelle 1 sind Versuchsergebnisse wiedergegeben:

**Tabelle 1**

| Verbindung | MR Antagonismus IC50 [nM] | MR Antagonismus Wirksamkeit [% des Maximaleffektes] | PR *in vivo* ED50 [mg/kg/d s.c.] |
|---|---|---|---|
| 17β-Cyano-15α,16α-methylen-19-nor-androst-4-en-3-on | 16,0 | 93,01 | 0,8 |
| 17β-Cyano-17α-methyl-15β,16β-methylen-19-nor-androst-4-en-3-on | 29,0 | 96,00 | 0.8 |
| 7α,17α-Bismethyl-17β-cyano-19-nor-androst-4-en-3-on | 31,0 | 96,20 | 1,0 |
| 17β-Cyano-15β, 16β-methylen-19-nor-androst-4-en-3-on | 4,5 | 95,57 | 0,84 |
| 17β-Cyano-17α-ethyl-15β,16β-methylen-19-nor-androst-4-en-3-on | 440 | 106,4 | 1,9 |
| 17β-Cyano-7α-methyl-19-nor-androst-4-en-3-on | 8,2 | 108,02 | 0,33 |
| 17β-Cyano-15β,16β-methylen-19-nor-androsta-4,6-dien-3-on | 15 | 108,51 | 3,3 |
| 17β-Cyano-17α-methyl-15β,16β-methylen-19-nor-androsta-4,6-dien-3-on | 190 | 114 | 2,1 |
| 17β-Cyano-7α-methyl-15β,16β-methylen-19-nor-androst-4-en-3-on | 8,3 | 108,4 | 0,11 |
| 17β-Cyano-7α-methyl-15β,16β-methylen-19-nor-androst-4-en-3-on | 10,3 | 110 | 2,9 |
| 17β-Cyano-17α-ethyl-6α,7α-methylen-15β,16β-methylen-19-nor-androst-4-en-3-on | 90,17 | 160 | 0,22 |

Die nachfolgenden Beispiele zur Synthese bevorzugter Verbindungen dienen zur weiteren Veranschaulichung der Erfindung. Die in den einzelnen Synthesebeispielen offenbarten neuen Zwischenprodukte sind ebenso wie die erfindungsgemäßen 17β-Cyano-19-nor-androst-4-en-Derivate erfindungswesentlich.

Viele der im Folgenden beschriebenen Umsetzungen führen zu Epimerengemischen. In der Regel wird die chromatographische Trennung dieser Gemische via präparativer HPLC unter folgenden Bedingungen durchgeführt: Trennungen wurden an chiraler Normalphase durchgeführt, wobei als stationäre Phase in der Regel Chiralpak AD-H 5µ Verwendung fand. Üblicherweise wurde mit einem Gemisch aus Hexan und Ethanol eluiert. In einigen Fällen fanden aber auch andere Eluentengemische, wie etwa Gemische aus Methanol und Ethanol, Verwendung:

### Beispiel 1

### 17β-Cyano-15β,16β-Methylen-19-nor-androst-4-en-3-on

### 1a.

### 15a-Acetoxy-19-nor-androst-4-en-3,17-dion

95 g 15α-Hydroxy-19-nor-androstendion (beschrieben in DE-A 24 56 068; 1976) wurden in 332 ml Pyridin gelöst. Nach Zusatz von 166 ml Acetanhydrid wurde drei Stunden bei Raumtemperatur gerührt. Dann wurde die Reaktionsmischung in ein Gemisch, bestehend aus 10 l Eiswasser, 109 ml konz. Schwefelsäure und 16 ml Methanol gegossen. Nach Rühren über Nacht wurde der Niederschlag abgesaugt und der Filterrückstand mit 3 Litern Wasser gewaschen. Man erhielt 15α-Acetoxy-19-nor-androst-4-en-3,17-dion, welches ohne Reinigung weiter umgesetzt wurde.

### 1b.

### 15α-Acetoxy-3-methoxy-19-nor-androsta-3,5-dien-17-on

90,6 g der in Beispiel 1a beschriebenen Verbindung wurden in 955 ml 2,2-Dimethoxypropan suspendiert und mit 10,3 g Pyridiniumtosylat versetzt. Nach 6,5-stündigem Erhitzen der Reaktionsmischung auf 100°C wurde über Nacht bei RT nachgerührt. Nach Zusatz von 13,8 ml Pyridin wurde am Rotationsverdampfer unter vermindertem Druck teilweise eingeengt und der verbliebene Kolbeninhalt mit 550 ml Methanol versetzt. Nach eineinhalbstündigem Rühren bei Raumtemperatur wurde auf 0°C gekühlt, abgesaugt und der Filterkuchen getrocknet. Man erhielt so 15α-Acetoxy-3-methoxy-19-nor-androsta-3,5-dien-17-on.

¹H-NMR (d6-DMSO): 0,87(s,3H,18-CH3), 1,98(s,3H,CH3-CO-), 3,46(s,3H,3-O-CH3), 5,10(m,1H,H-15), 5,18(s,1H,H-4), 5,21(m,1H,H-6)

### 1 c.

### 15β,16β-Methylen-3-methoxy-19-nor-androsta-3,5-dien-17-on

26,03 g Trimethylsulfoxoniumiodid und 8,3 g gepulvertes Natriumhydroxyd wurden in 344 ml Dimethylsulfoxid 30 Minuten bei 50°C Badtemperatur gerührt. Die so erhaltene Lösung wurde innerhalb 5 Minuten zu einer Suspension von 33,4 g der in Beispiel 1 b beschriebenen Verbindung in 110 ml Dimethylsulfoxid zugetropft. Nach 20 Minuten wurde der Ansatz in ein Becherglas überführt und unter Rühren wurden langsam 500 ml Wasser zugetropft. Nachdem 20 Minuten gerührt worden war, wurde über eine Fritte abgesaugt und der Filterkuchen getrocknet. Man erhielt 15β,16β-Methylen-3-methoxy-19-nor-androsta-3,5-dien-17-on.

¹H-NMR (d6-DMSO): 0,91(s,3H,18-CH3), 3,51(s,3H,3-O-CH3), 5,26(s,1H,H-4), 5,33(m,1H,H-6)

### 1d.

### 17-Cyano-15β,16β-Methylen-3-methoxy-19-nor-androsta-3,5-dien

2,5 g der in Beispiel 1 c beschriebenen Verbindung wuden in einem Gemisch aus 40 ml 1,2-Dimethoxyethan und 25 ml tertiär-Butanol vorgelegt. Nach Eintragen von 4,7 g Kaliumtertiärbutylat wuden 2,77 g Tosylmethylisocyanid (TOSMIC) zugegeben, und es wurde 90 Minuten nachgerührt. Der Ansatz wurde auf die zehnfache Menge Eiswasser gegeben, Kochsalz wurde bis zu Sättigung zugegeben und filtriert. Der Filterkuchen wurde in Ethylacetat aufgenommen, mit Wasser und Kochsalzlösung gewaschen, über Natriumsulfat getrocknet, filtriert und das Filtrat wurde eingeengt. Man erhielt ein Gemisch aus 17α-Cyano- und 17β-Cyano-15β,16β-Methylen-3-methoxy-19-nor-androsta-3,5-dien, welches ohne Reinigung weiter umgesetzt wurde.

### 1e.

### 17β-Cyano-15β,16β-Methylen-19-nor-androst-4-en-3-on

2,8 g des in Beispiel 1d beschriebenen rohen Isomerengemisches wurden 3 Stunden in einer Mischung aus 100 ml Aceton und 10 ml 1 normaler Salzsäure gerührt. Nach Neutralisation der Reaktionsmischung mit gesättigter Natriumhydrogencarbonatlösung wurde mit Ethylacetat extrahiert und die organische Phase anschließend mit Wasser und gesättigter Kochsalzlösung gewaschen. Nach Trocknung über Natriumsulfat wurde filtriert, das Filtrat eingeengt und der Rückstand an Kieselgel zunächst mit einem Gradienten aus den Lösemitteln n-Hexan und Ethylacetat chromatographiert. Die produkthaltigen Fraktionen wurden dann an Kieselgel mit einem Gemisch aus n-Hexan und Ethylacetat erneut chromatographiert.

Die überwiegend das gewünschte Produkt enthaltenden Fraktionen wurden zusammengefasst, eingeengt und aus einem Gemisch von Diisopropylether und Aceton umkristallisiert. Man erhielt als Kristallisat 17β-Cyano-15β,16β-Methylen-19-nor-androst-4-en-3-on. Die Mutterlauge und die restlichen produkthaltigen Fraktionen der Chromatographie ergaben nach dem Einengen ein Gemisch von 17α-Cyano- und 17β-Cyano-15β,16β-Methylen-19-nor-androst-4-en-3-on.

¹H-NMR (300 MHz, CDCl₃ TMS als interner Standard, ausgewählte Signale): δ = 0,46(m,1H), 0,90(m,1H), 1,04(m,1H), 1,10(s,3H,18-CH3), 1,67(m,1H), 1,86(m,2H), 2,11(m,2H), 2,55(m,1H), 2,77(d breit,1H,J=4,4Hz,17-H), 5,86(s,1H, H-4)

### Beispiel 2

### 17β-Cyano-15β,16β-methylen-19-nor-androsta-4,6-dien-3-on

### 2a.

### 17β-Cyano-15β,16β-methylen 3-methoxy-19-nor-androsta-3,5-dien

9g 17β-Cyano-15β,16β-Methylen-19-nor-androst-4-en-3-on (siehe Beispiel 1 e), gelöst in 92 ml Methanol, wurden mit 83 ml Orthoameisensäuremethylester versetzt. Nach Zugabe von 53 mg p-Toluolsulfonsäure wurde bei 15°C gerührt. Es bildete sich ein Niederschlag. Nach Zusatz von 0,8 ml Pyridin bei 0°C wurde auf -10°C gekühlt und 30 Minuten nachgerührt. Nach Einengen unter vermindertem Druck wurde 17β-Cyano-15β,16β-methylen 3-methoxy-19-nor-androsta-3,5-dien erhalten, welches ohne Reinigung weiter umgesetzt wurde.

### 2b.

### 17β-Cyano-15β,16β-methylen-19-nor-androsta-4,6-dien-3-on

Eine Suspension von 3,4 g 17β-Cyano-15β,16β-methylen 3-methoxy-19-nor-androsta-3,5-dien in 100 ml 1-Methyl-2-pyrrolidon wurde nacheinander bei 0°C mit 4 ml einer 10%igen Natriumacetatlösung sowie bei dieser Temperatur mit 1,6 g 1,3-Dibrom-5,5-dimethylhydantoin portionsweise versetzt, 0,5 Stunden bei 0°C (Eisbad) gerührt, mit 1,5 g Lithiumbromid sowie 1,3 g Lithiumcarbonat versetzt, und 3,5 Stunden bei 100°C Badtemperatur gerührt. Anschließend rührte man in Eiswasser /Kochsalz ein, filtrierte den Niederschlag ab. Man erhielt 17β-Cyano-15β,16β-methylen-19-nor-androsta-4,6-dien-3-on.

¹H-NMR (300 MHz, CDCl₃ TMS als interner Standard, ausgewählte Signale): δ = 0,54(m,1H, cyclopropyl) 1,10 (m,1H, cyclopropyl), 1,12(s,3H, 18-CH3), 2,80(d,1H,J=4,4, H-17), 5.81(s,1H, H-4), 6,27(m,1H, H-6), 6,41(m,1H,H-7)

### Beispiel 3

### 17β-Cyano-15β,16β-methylen-7α-methyl-19-nor-androst-4-en-3-on und 17β-Cyano-15β,16β-methylen-7β-methyl-19-nor-androst-4-en-3-on

Zu einer Lösung von 1,0 g 17β-Cyano-15β,16β-methylen-19-nor-androsta-4,6-dien-3-on in 50 ml Tetrahydrofuran gab man bei Raumtemperatur 67 mg Kupfer-I-chlorid und rührte 10 Minuten, bevor man auf -15°C abkühlte, mit 450 mg Aluminiumchlorid versetzte, 30 Minuten bei dieser Temperatur rührte, mit 4,5 ml Methylmagnesiumbromidlösung (3 M in Tetrahydrofuran) tropfenweise versetzte, und eine Stunde bei -15°C rührte. Zur Aufarbeitung wurde die Reaktionsmischung bei -15°C mit 30 ml 2 M Salzsäure versetzt, 0,5 Stunden bei Raumtemperatur gerührt, auf Wasser gegeben, dreimal mit Essigester extrahiert, über Natriumsulfat getrocknet, im Vakuum eingeengt, und an Kieselgel mit Hexan / Ethylacetat chromatographiert. Man erhielt 17β-Cyano-7α-methyl-18α-homo-19-nor-androst-4-en-3-on als Fraktion I und als Fraktion II 17β-Cyano-15β,16β-methylen-7β-methyl-19-nor-androst-4-en-3-on.

### 17β-Cyano-15β,16β-methylen-7α-methyl-19-nor-androst-4-en-3-on:

¹H-NMR (300 MHz, CDCl₃ TMS als interner Standard, ausgewählte Signale): δ = 0,45 (m,1H,cyclopropyl) 0,88 (d,3H,J=6,97, 7-CH3), 1,03(m,1H,cyclopropyl) 1,10(s,3H, 18-CH3), 5,86(s,1H, H-4)

### 17β-Cyano-15β,16β-methylen-7β-methyl-19-nor-androst-4-en-3-on:

¹H-NMR (300 MHz, CDCl₃ TMS als interner Standard, ausgewählte Signale): δ = 0,53 (m,1H,cyclopropyl) 1,01 (m,1H,cyclopropyl) 1,10(s,3H, 18-CH3), 1,21 (d,3H,J=6,22, 7-CH3), 5,83(s,1H, H-4)

### Beispiel 4

### 17β-Cyano-7α-ethyl-15β,16β-methylen-19-nor-androst-4-en-3-on und 17β-Cyano-7β-ethyl-15β,16β-methylen-19-nor-androst-4-en-3-on

Nach der Methode des Beispiels 3 mit Ethylmagnesiumbromid in Ether anstelle des Methylmagnesiumbromids erhielt man nach der Chromatographie als Fraktion I 17β-Cyano-7α-ethyl-15β,16β-methylen-19-nor-androst-4-en-3-on und als Fraktion II 17β-Cyano-7β-ethyl-15β, 16β-methylen-19-nor-androst-4-en-3-on.

### 17β-Cyano-7α-ethyl-15β,16β-methylen-19-nor-androst-4-en-3-on:

¹H-NMR (300 MHz, CDCl₃ TMS als interner Standard, ausgewählte Signale): δ = 0,46 (m,1H,cyclopropyl) 0,92 (m,3H, 7-CH3-CH2); 1,03(m,1H,cyclopropyl) 1,10(s,3H, 18-CH3), 5,87(s,1H, H-4)

### 17β-Cyano-7β-ethyl-15β,16β-methylen-19-nor-androst-4-en-3-on:

¹H-NMR (300 MHz, CDCl₃ TMS als interner Standard, ausgewählte Signale): δ = 0,54 (m,1H,cyclopropyl) 0,95 (m,3H, 7-CH3-CH2), 1,02(m,1H,cyclopropyl) 1,11(s,3H, 18-CH3); 5,84(s,1H, H-4)

### Beispiel 5

### 17β-Cyano-7α-vinyl-15β,16β-methylen-19-nor-androst-4-en-3-on und 17β-Cyano-7β-vinyl-15β,16β-methylen-19-nor-androst-4-en-3-on

Nach der Methode des Beispiels 3 mit Vinylmagnesiumbromid anstelle des Methylmagnesiumbromids erhielt man nach der Chromatographie als Fraktion I 17β-Cyano-7α-vinyl-15β,16β-methylen-19-nor-androst-4-en-3-on und als Fraktion II 17β-Cyano-7β-vinyl-15β,16β-methylen-19-nor-androst-4-en-3-on.

### 17β-Cyano-7α-vinyl-15β,16β-methylen-19-nor-androst-4-en-3-on:

¹H-NMR (300 MHz, CDCl₃ TMS als interner Standard, ausgewählte Signale): δ = 0,51 (m,1H,cyclopropyl), 1,08(m,1H,cyclopropyl) 1,14(s,3H, 18-CH3),5,22(m,2H, CH2=CH), 5,88 (m,1H,CH2=CH) 5,92(s,1H, H-4)

### 17β-Cyano-7β-vinyl-15β,16β-methylen-19-nor-androst-4-en-3-on:

¹H-NMR (300 MHz, CDCl₃ TMS als interner Standard, ausgewählte Signale): δ = 0,42 (m,1H,cyclopropyl), 0,95(m,1H,cyclopropyl) 1,10(s,3H, 18-CH3),5,05(m,2H, CH2=CH), 5,86(s,1H, H-4), 5,88 (m,1H,CH2=CH)

### Beispiel 6

### 17β-Cyano-7α-cyclopropyl-15β,16β-methylen-19-nor-androst-4-en-3-on und 17β-Cyano-7β-cyclopropyl-15β,16β-methylen-19-nor-androst-4-en-3-on

Nach der Methode des Beispiels 3 mit Cyclopropylmagnesiumbromid anstelle des Methylmagnesiumbromids erhielt man nach der Chromatographie als Fraktion I 17β-Cyano-7α-cyclopropyl-15β,16β-methylen-19-nor-androst-4-en-3-on und als Fraktion II 17β-Cyano-7β-cyclopropyl-15β,16β-methylen-19-nor-androst-4-en-3-on.

### 17β-Cyano-7α-cyclopropyl-15β,16β-methylen-19-nor-androst-4-en-3-on:

¹H-NMR (300 MHz, CDCl₃ TMS als interner Standard, ausgewählte Signale): δ = - 0,05(m,1H,cyclopropyl), 0,26(m,1H,cyclopropyl), 0,47(m,3H, cyclopropyl), 1,08(s,3H, 18-CH3), 5,90(s,1H, H-4)

### 17β-Cyano-7β-cyclopropyl-15β,16β-methylen-19-nor-androst-4-en-3-on:

¹H-NMR (300 MHz, CDCl₃ TMS als interner Standard, ausgewählte Signale): δ = 0,29(m,2H,cyclopropyl), 0,47(m,1H,cyclopropyl), 0,60(m,2H, cyclopropyl), 0,78(m,1H, cyclopropyl), 0,97(m,3H, cyclopropyl), 1,12(s,3H, 18-CH3), 5,81(s,1H, H-4)

### Beispiel 7

### 17β-Cyano-6β-hydroxymethyl-15β,16β-methylen-19-nor-androst-4-en-3-on

3 g 17β-Cyano-15β,16β-methylen-19-nor-androst-4-en-3-on (siehe Beispiel 1e) wurden in 16 ml Methanol aufgenommen, mit 1,6 ml Pyrrolidin versetzt und 1 h am Rückfluss erwärmt. Nach dem Abkühlen wurde abgesaugt, mit wenig kaltem Methanol nachgewaschen und trocken gezogen. Das Kristallisat wurde in 30 ml Benzol und 60 ml Ethanol gelöst, 3,1 ml 30 %ige Formaldehydlösung wurden zugegeben. Nach 2h Rühren bei Raumtemperatur wurde zur Trockene eingeengt und an Kieselgel chromatographiert. Man erhielt 17β-Cyano-6β-hydroxymethyl-15β,16β-methylen-19-nor-androst-4-en-3-on.

¹H-NMR (300 MHz, CDCl₃ TMS als interner Standard, ausgewählte Signale): δ = 1,09(s,3H, 18-CH3), 0,43-1,06(m,2H,cyclopropyl), 3,74(m,2H,CH2OH) 5,94(s,1H, H-4)

### Beispiel 8

### 17β-Cyano-6,6-ethylen-15β,16β-methylen-19-nor-androst-4-en-3-on

### 8a.

### 17β-Cyano-15β,16β-methylen -6β-tosyloxymethyl -19-nor-androst-4-en-3-on

Zu einer Lösung von 1,74 g 17β-Cyano-6β-hydroxymethyl-15β,16β-methylen-19-nor-androst-4-en-3-on in 20 ml Pyridin wurden in einer Portion 2,93 g para-Toluolsulfonsäurechlorid gegeben und 6 Stunden bei Raumtemperatur gerührt. Danach wurde die Reaktionsmischung in eiskalte 1 N HCl gegossen, das ausgefallene Rohprodukt abgesaugt und erneut in Ethylacetat gelöst. Nach dem jeweils zweimaligem Waschen mit Wasser, ges. Bicarbonatlösung und ges. Kochsalzlösung sowie dem Trocknen der organischen Phase mit Natriumsulfat erhielt man nach dem Einengen zur Trockne 17β-Cyano-15β,16β-methylen -6β-tosyloxymethyl -19-nor-androst-4-en-3-on, das ohne Reinigung weiterverwendet wurde.

### 8b.

### 17β-Cyano-6,6-ethylen-15β,16β-methylen-19-nor-androst-4-en-3-on

Zu einer Lösung von 3 g Trimethylsulfoxoniumiodid in 50 ml trockenem DMSO wurden bei Raumtemperatur portionsweise 450 mg Natriumhydrid gegeben und nach beendeter Zugabe 1 Stunde bei Raumtemperatur gerührt. Anschließend wurde die Lösung von 1,5 g 17β-Cyano-15β,16β-methylen -6β-tosyloxymethyl-19-nor-androst-4-en-3-on zum gebildeten Ylid gegeben und 6 Stunden bei Raumtemperatur nachgerührt. Nach dem Abbruch der Reaktion durch die Zugabe von 350 ml Wasser, zweimaliger Extraktion mit 150 ml Essigester, Wäsche der organischen Phase mit Wasser und gesättigter Kochsalzlösung sowie dem Trocknen über Natriumsulfat wurde die organische Phase eingeengt, und man erhielt 17β-Cyano-6,6-ethylen-15β,16β-methylen-19-nor-androst-4-en-3-on.

¹H-NMR (300 MHz, CDCl₃ TMS als interner Standard, ausgewählte Signale): δ = 0,39-1,02(m,6H,6,6-ethylen/cyclopropyl) 1,11 (s,3H,18-CH3) 5,70(s,1H, H-4)

### Beispiel 9

### 17β-Cyano-6β,7β-methylen-15β,16β-methylen-19-nor-androst-4-en-3-on und 17β-Cyano-6α,7α-methylen-15β,16β-methylen-19-nor-androst-4-en-3-on

Zu einer Lösung von 3,93 g Trimethylsulfoxoniumiodid in 38 ml trockenem Dimethylsulfoxid wurden bei Raumtemperatur portionsweise 714 mg Natriumhydrid (60%-ig in Paraffin) gegeben und nach beendeter Zugabe 1 Stunde bei Raumtemperatur gerührt. Anschließend wurde die Lösung von 2,0 g 17β-Cyano-15β,16β-methylen-19-nor-androsta-4,6-dien-3-on in Dimethylsulfoxid zum gebildeten Ylid gegeben und 6 Stunden bei Raumtemperatur nachgerührt. Nach dem Abbruch der Reaktion durch Zugabe von 150 ml Ammoniumchloridlösung, zweimalige Extraktion mit 75 ml Essigsäureethylester, Wäsche der organischen Phase mit Wasser und gesättigter Kochsalzlösung sowie Trocknen über Natriumsulfat wurde die organische Phase zur Trockne eingeengt. Nach Flash-Chromatographie an Kieselgel mit Hexan / Essigsäureethylester und anschließener HPLC-Trennung an chiraler stationärer Normalphase mit einem Eluenten aus Hexan und Ethanol wurden 17β-Cyano-6β,7β-methylen-15β,16β-methylen-19-nor-androst-4-en-3-on als Fraktion I und 17β-Cyano-6α,7α-methylen-15β,16β-methylen-19-nor-androst-4-en-3-on als Fraktion II erhalten.

### 17β-Cyano-6β,7β-methylen-15β,16β-methylen-19-nor-androst-4-en-3-on:

¹H-NMR (300 MHz, CDCl₃ TMS als interner Standard, ausgewählte Signale): δ = 0,46-0,62(2 x m,2H,cyclopropyl) 1,06(s,3H,18-CH3), 2,79(d,1H,J=4,14, H-17), 6,12(s,1H, H-4)

### 17β-Cyano-6α,7α-methylen-15β,16β-methylen-19-nor-androst-4-en-3-on:

¹H-NMR (300 MHz, CDCl₃ TMS als interner Standard, ausgewählte Signale): δ = 0,49, 0,77, 0,83, 0,98(4 x m,4H,cyclopropyl) 1,11(s,3H,18-CH3), 2,77(d,1H,J=4,40, H-17), 6,05(s,1H, H-4)

### Beispiel 10

### 17β-Cyano-17α-methyl-15β,16β-methylen-19-nor-androst-4-en-3-on

### 10a.

### 17β-Cyano-17a-methyl-15β,16β-methylen 3-methoxy-19-nor-androsta-3,5-dien

Zu einer Lösung von 2,6 g 17β-Cyano-15β,16β-methylen 3-methoxy-19-nor-androsta-3,5-dien in 80 ml THF wurden bei -78°C 14,7 ml 2 M Lithiumdiisopropylamidlösung getropft. Es wurde 1 Stunde bei -78°C gerührt, 2,35 ml Methyliodid wurden zugegeben und dann wurde auf Raumtemperatur erwärmt. 25 ml gesättigte Ammoniumchlorid wurden zugeben, und es wurde mit dreimal 100 ml Essigsäureethylester extrahiert. Die vereinigten organischen Extrakte wurden eingeengt und aus Methanol kristallisiert. Man erhielt 17β-Cyano-17α-methyl-15β,16β-methylen 3-methoxy-19-nor-androsta-3,5-dien, welches sofort weiter umgesetzt wurde.

### 10b.

### 17β-Cyano-17α-methyl-15β,16β-methylen-19-nor-androst-4-en-3-on

2g 17β-Cyano-17α-methyl-15β,16β-methylen 3-methoxy-19-nor-androsta-3,5-dien wurden in 50 ml Methanol aufgenommen, mit 3 ml 1 N Salzsäure versetzt. Nach 1 Stunde wurde mit gesättigter Natriumhydrogencarbonatlösung neutralisiert und im Vakuum eingeengt wobei das Produkt ausfiel. Es wurde abgesaugt, mit Wasser gewaschen und aus Essisäureethylester umkristallisiert. Man erhielt 17β-Cyano-17α-methyl-15β,16β-methylen-19-nor-androst-4-en-3-on.

¹H-NMR (300 MHz, CDCl₃ TMS als interner Standard, ausgewählte Signale): δ = 0,42(m,1H, cyclopropyl) 0,86 (m,1H, cyclopropyl), 1,06 (m,1H, cyclopropyl), 1,18(s,3H, 18-CH3), 1,37(s,3H, 17-CH3), 5,84(s,1H, H-4)

### Beispiel 11

### 17β-Cyano-6β-hydroxymethyl-19-nor-androst-4-en-3-on

17β-Cyano-19-nor-androst-4-en-3-on wurde analog der in Beispiel 8 angegeben Verfahrensweise umgesetzt. Man erhielt 17β-Cyano-6β-hydroxymethyl-19-nor-androst-4-en-3-on.

¹H-NMR (300 MHz, CDCl₃ TMS als interner Standard, ausgewählte Signale): δ = 0,97(s,3H, 18-CH3), 3,66(m,2H,CH2OH) 5,91(s,1H, H-4)

### Beispiel 12

### 17β-Cyano-6,6-ethyliden-19-nor-androst-4-en-3-on

17β-Cyano-6β-hydroxymethyl-19-nor-androst-4-en-3-on wurde analog den in den Beispielen 8a und 8b angegebenen Vorschriften umgesetzt. Man erhielt so 17β-Cyano-6,6-ethyliden-1 9-nor-androst-4-en-3-on.

¹H-NMR (300 MHz, CDCl₃ TMS als interner Standard, ausgewählte Signale): δ = 0,41 (m,1H), 0,54 (m,1H), 0,68 (m,1H), 1,01 (s,3H,18-CH3), 2,45 (s breit,1H), 5,69 (s,1H, H-4)

### Beispiel 13

### 17β-Cyano-19-nor-androsta-4,6-dien-3-on

### 13a.

### 17β-Cyano-3-methoxy-19-nor-androsta-3,5-dien

17β-Cyano-19-nor-androst-4-en-3-on wurde analog der in Vorschrift 2a angegebenen Methode umgesetzt. Man erhielt 17β-Cyano-3-methoxy-19-nor-androsta-3,5-dien.

¹H-NMR (d6-DMSO): 0,81(s,3H, 18-CH3), 3,45(s,3H,OCH3), 5,19(s breit,2H, H-4 und H-6)

### 13b.

### 17β-Cyano-19-nor-androsta-4,6-dien-3-on

17β-Cyano-3-methoxy-19-nor-androsta-3,5-dien wurde analog der in Vorschrift 2b angegebenen Methode umgesetzt. Man erhielt 17β-Cyano-19-nor-androsta-4,6-dien-3-on.

¹H-NMR (d6-DMSO): 0,86(s,3H, 18-CH3), 2,80(d,1H,J=4,4, H-17), 5,69(s,1H, H-4), 6,18(m,1H, H-6), 6,24(m,1H,H-7)

### Beispiel 14

### 17β-Cyano-6β, 7β-methylen-19-nor-androst-4-en-3-on und 17β-Cyano-6α, 7α-methylen-19-nor-androst-4-en-3-on

17β-Cyano-19-nor-androsta-4,6-dien-3-on wurde analog der in Beispiel 9 angegebenen Methode umgesetzt. Es wurden 17β-Cyano-6β, 7β-methylen-19-nor-androst-4-en-3-on und 17β-Cyano-6a,7a-methylen-19-nor-androst-4-en-3-on erhalten.

### 17β-Cyano-6β, 7β-methylen-19-nor-androst-4-en-3-on:

¹H-NMR (300 MHz, CDCl₃ TMS als interner Standard, ausgewählte Signale): δ = 0,52 (m,1H), 0,97 (m,1H), 0,97(s,3H,18-CH3), 6,11 (s,1H, H-4)

### 17β-Cyano-6α,7α-methylen-19-nor-androst-4-en-3-on:

¹H-NMR (300 MHz, CDCl₃ TMS als interner Standard, ausgewählte Signale): δ = 0,66 (m,1H), 0,78 (m,1H), 0,89 (m,1H), 1,01(s,3H,18-CH3), 6,03 (s,1H, H-4)

### Beispiel 15

### 17β-Cyano-7α-methyl-19-nor-androst-4-en-3-on und 17β-Cyano-7β-methyl-19-nor-androst-4-en-3-on

17β-Cyano-19-nor-androsta-4,6-dien-3-on wurde analog der in Beispiel 3 angegebenen Methode umgesetzt. Es wurden 17β-Cyano-7α-methyl-19-nor-androst-4-en-3-on und 17β-Cyano-7β-methyl-19-nor-androst-4-en-3-on erhalten.

### 17β-Cyano-7α-methyl-19-nor-androst-4-en-3-on:

¹H-NMR (300 MHz, CDCl₃ TMS als interner Standard, ausgewählte Signale): δ = 0,77 (d,3H, 7-CH3, J=7Hz), 1,00(s,3H,18-CH3), 5,84 (s,1H, H-4)

### 17β-Cyano-7β-methyl-19-nor-androst-4-en-3-on:

¹H-NMR (300 MHz, CDCl₃ TMS als interner Standard, ausgewählte Signale): δ = 0,78 (d,3H, 7-CH3, J=7 Hz), 1,00(s,3H,18-CH3), 5,85 (s,1H, H-4)

### Beispiel 16

### 17β-Cyano-7α-ethyl-19-nor-androst-4-en-3-on und 17β-Cyano-7β-ethyl-19-nor-androst-4-en-3-on

17β-Cyano-19-nor-androsta-4,6-dien-3-on wurde analog der in Beispiel 3 angegebenen Methode umgesetzt, wobei mit Ethylmagnesiumbromid in Diethylether statt mit Methylmagnesiumbromid gearbeitet wurde. Es wurden 17β-Cyano-7α-ethyl-19-nor-androst-4-en-3-on und 17β-Cyano-7β-ethyl-19-nor-androst-4-en-3-on erhalten.

### 17β-Cyano-7α-ethyl-19-nor-androst-4-en-3-on:

¹H-NMR (d6-DMSO): 0,80 (t,3H, 7-CH2-CH3, J=7,5Hz), 0,87(s,3H,18-CH3), 5,73 (s,1H, H-4)

### 17β-Cyano-7β-ethyl-19-nor-androst-4-en-3-on:

¹H-NMR (300 MHz, CDCl₃ TMS als interner Standard, ausgewählte Signale): δ = 0,88 (t,3H, 7-CH2-CH3, J=7,5Hz), 1,00(s,3H,18-CH3), 5,82 (s,1H, H-4)

### Beispiel 17

### 17β-Cyano-7α-vinyl-19-nor-androst-4-en-3-on und 17β-Cyano-7β-vinyl-19-nor-androst-4-en-3-on

17β-Cyano-19-nor-androsta-4,6-dien-3-on wurde analog der in Beispiel 3 angegebenen Methode umgesetzt, wobei mit Vinylmagnesiumbromid statt mit Methylmagnesiumbromid gearbeitet wurde. Es wurden 17β-Cyano-7α-vinyl-19-nor-androst-4-en-3-on und 17β-Cyano-7β-vinyl-19-nor-androst-4-en-3-on erhalten.

### 17β-Cyano-7α-vinyl-19-nor-androst-4-en-3-on:

¹H-NMR (300 MHz, CDCl₃ TMS als interner Standard, ausgewählte Signale): δ = 0,99(s,3H, 18-CH3), 5,10(m,2H, CH2=CH), 5,70 (m,1H,CH2=CH), 5,85 (s,1H, H-4)

### 17β-Cyano-7β-vinyl-19-nor-androst-4-en-3-on:

¹H-NMR (300 MHz, CDCl₃ TMS als interner Standard, ausgewählte Signale): δ = 0,99(s,3H, 18-CH3), 4,94(d breit,1H,J=10Hz, CH2=CH), 5,04(d breit, 1H,J=17Hz, CH2=CH), 5,71 (m,1H,CH2=CH), 5,84 (s,1H, H-4)

### Beispiel 18

### 17β-Cyano-7a-cyclopropyl-19-nor-androst-4-en-3-on und 17β-Cyano-7β-cyclopropyl-19-nor-androst-4-en-3-on

17β-Cyano-19-nor-androsta-4,6-dien-3-on wurde analog der in Beispiel 3 angegebenen Methode umgesetzt, wobei mit Cyclopropylmagnesiumbromid statt mit Methylmagnesiumbromid gearbeitet wurde. Es wurden 17β-Cyano-7α-cyclopropyl-19-nor-androst-4-en-3-on und 17β-Cyano-7β-cyclopropyl-19-nor-androst-4-en-3-on erhalten.

### 17β-Cyano-7α-cyclopropyl-19-nor-androst-4-en-3-on:

¹H-NMR (300 MHz, CDCl₃ TMS als interner Standard, ausgewählte Signale): δ = - 0,05(m,1H,cyclopropyl), 0,27(m,1H,cyclopropyl), 0,47(m,3H, cyclopropyl), 1,00(s,3H, 18-CH3), 5,88(s,1H, H-4)

### 17β-Cyano-7β-cyclopropyl-19-nor-androst-4-en-3-on:

¹H-NMR (300 MHz, CDCl₃ TMS als interner Standard, ausgewählte Signale): δ = 0,13(m,1H,cyclopropyl), 0,29(m,1H,cyclopropyl), 0,58(m,4H, cyclopropyl), 1,01(s,3H, 18-CH3), 5,81(s,1H, H-4)

### Beispiel 19

### 17α-Allyl-17β-cyano-19-nor-androst-4-en-3-on

17β-Cyano-3-methoxy-19-nor-androsta-3,5-dien wurde analog den in den Beispielen 10a (wobei Allylbromid statt Methyliodid verwendet wurde) und 10b angegebenen Methoden umgesetzt. Man erhielt 17α-Allyl-17β-cyano-19-nor-androst-4-en-3-on.

¹H-NMR (300 MHz, CDCl₃ TMS als interner Standard, ausgewählte Signale): δ = 0,85(m,1H), 1,15(s,3H, 18-CH3), 5,22 (m,2H, -CH=CH2), 5,84(s,1H, H-4), 5,92 (m,1H, -CH=CH2)

### Beispiel 20

### 17β-Cyano-17α-ethyl-19-nor-androst-4-en-3-on

17β-Cyano-3-methoxy-19-nor-androsta-3,5-dien wurde analog den in den Beispielen 10a (wobei Ethyliodid statt Methyliodid verwendet wurde) und 10b angegebenen Methoden umgesetzt. Man erhielt 17β-Cyano-17α-ethyl-19-nor-androst-4-en-3-on.

¹H-NMR (d6-DMSO): 0,97(t,3H, 17-CH2-CH3), 1,00(s,3H, 18-CH3), 5,69 (m,1H, - CH=CH2)

### Beispiel 21

### 17β-Cyano-17α-methyl-19-nor-androst-4-en-3-on

### 21a.

### 17β-Cyano-17α-methyl-3-methoxy-19-nor-androsta-3,5-dien

17β-Cyano-3-methoxy-19-nor-androsta-3,5-dien wurde analog der im Beispiel 10a angegebenen Methode umgesetzt. Man erhielt 17β-Cyano-17α-methyl-3-methoxy-19-nor-androsta-3,5-dien.

¹H-NMR (d6-DMSO): 0,93(s,3H), 1,20(s,3H), 3,45 (s,3H, 3-O-CH3), 5,19 (m,2H, H4 und H6)

### 21 b.

### 17β-Cyano-17α-methyl-19-nor-androst-4-en-3-on

17β-Cyano-17α-methyl-3-methoxy-19-nor-androsta-3,5-dien wurde analog der im Beispiel 10b angegebenen Methode umgesetzt. Man erhielt 17β-Cyano-17α-methyl-19-nor-androst-4-en-3-on.

¹H-NMR (d6-DMSO): 0,97(s,3H), 1,19(s,3H), 5,69 (s,1H, H-4)

### Beispiel 22

### 17β-Cyano-6β-hydroxymethyl-17α-methyl-19-nor-androst-4-en-3-on

17β-Cyano-17α-methyl-19-nor-androst-4-en-3-on wurde analog der in Beispiel 8 angegebenen Verfahrensweise umgesetzt. Man erhielt 17β-Cyano-6β-hydroxymethyl-17α-methyl-19-nor-androst-4-en-3-on.

¹H-NMR (300 MHz, CDCl₃ TMS als interner Standard, ausgewählte Signale): δ = 1,09(s,3H), 1,29(s,3H), 3,68 (m,2H, 6-CH2-OH), 5,91 (s,1H, H-4)

### Beispiel 23

### 17β-Cyano-6,6-ethyliden-17α-methyl-19-nor-androst-4-en-3-on

17β-Cyano-6β-hydroxymethyl-17α-methyl-19-nor-androst-4-en-3-on wurde analog den in den Beispielen 8a und 8b angegebenen Vorschriften umgesetzt. Man erhielt 17β-Cyano-6,6-ethyliden-17α-methyl-19-nor-androst-4-en-3-on.

¹H-NMR (300 MHz, CDCl₃ TMS als interner Standard, ausgewählte Signale): δ = 0,40(m,1H), 0,54(m,1H), 0,68(m,1H), 0,94(m,2H), 1,11(s,3H), 1,29(s,3H), 5,68(s,1H, H-4)

### Beispiel 24

### 17β-Cyano-17α-methyl-19-nor-androsta-4,6-dien-3-on

17β-Cyano-3-methoxy-19-nor-androsta-3,5-dien wurde analog der im 2b angegebenen Methode umgesetzt. Man erhielt 17β-Cyano-17α-methyl-19-nor-androsta-4,6-dien-3-on.

¹H-NMR (d6-DMSO): 1,04(s,3H), 1,25(s,3H), 5,73(s,1H, H-4), 6,23(m,1H, H-6), 6,29 (m,1H, H-7)

### Beispiel 25

### 17β-Cyano-7α,17α-bismethyl-19-nor-androst-4-en-3-on

17β-Cyano-17α-methyl-19-nor-androsta-4,6-dien-3-on wurde analog der in Beispiel 3 angegebenen Methode umgesetzt. Man erhielt 17β-Cyano-7α,17α-bismethyl-19-nor-androst-4-en-3-on.

¹H-NMR (300 MHz, CDCl₃ TMS als interner Standard, ausgewählte Signale): δ = 0,78(d,3H,J=7Hz,7-CH3), 1,11(s,3H), 1,31 (s,3H), 5,84(s,1H, H-4)

### Beispiel 26

### 17β-Cyano-17α-methyl-7α-vinyl-19-nor-androst-4-en-3-on und 17β-Cyano-17α-methyl-7β-vinyl-19-nor-androst-4-en-3-on

17β-Cyano-17α-methyl-19-nor-androsta-4,6-dien-3-on wurde analog der in Beispiel 3 angegebenen Methode umgesetzt, wobei mit Vinylmagnesiumbromid statt mit Methylmagnesiumbromid gearbeitet wurde. Es wurden 17β-Cyano-17α-methyl-7α-vinyl-19-nor-androst-4-en-3-on und 17β-Cyano-17α-methyl-7β-vinyl-19-nor-androst-4-en-3-on erhalten.

### 17β-Cyano-17α-methyl-7α-vinyl-19-nor-androst-4-en-3-on:

¹H-NMR (300 MHz, CDCl₃ TMS als interner Standard, ausgewählte Signale): δ = 1,11(s,3H), 1,24-1,31(m,8H), 5,10(m,2H,7-CH=CH2), 5,70(m,1H,7-CH=CH2), 5,89(s,1H, H-4)

### 17β-Cyano-17α-methyl-7β-vinyl-19-nor-androst-4-en-3-on:

¹H-NMR (300 MHz, CDCl₃ TMS als interner Standard, ausgewählte Signale): δ = 1,09(s,3H), 1,26(s,3H), 4,93(d breit, 1H,J=10Hz, 7-CH=CH2), 5,03(d breit, 1H,J=17Hz, 7-CH=CH2), 5,71(m,1H,7-CH=CH2), 5,83(s,1H, H-4)

### Beispiel 27

### 17β-Cyano-7α-cyclopropyl-17α-methyl-19-nor-androst-4-en-3-on und 17β-Cyano-7β-cyclopropyl-17α-methyl-19-nor-androst-4-en-3-on

17β-Cyano-17α-methyl-19-nor-androsta-4,6-dien-3-on wurde analog der in Beispiel 3 angegebenen Methode umgesetzt, wobei mit Cyclopropylmagnesiumbromid statt mit Methylmagnesiumbromid gearbeitet wurde. Es wurden 17β-Cyano-7α-cyclopropyl-17α-methyl-19-nor-androst-4-en-3-on und 17β-Cyano-7β-cyclopropyl-17α-methyl-19-nor-androst-4-en-3-on erhalten.

### 17β-Cyano-7α-cyclopropyl-17α-methyl-19-nor-androst-4-en-3-on:

¹H-NMR (300 MHz, CDCl₃ TMS als interner Standard, ausgewählte Signale): δ = - 0,05(m,1H), 0,26(m,1H), 0,39-0,58(m,3H), 1,10(s,3H), 1,32(s,3H), 5,89(s,1H, H-4)

### 17β-Cyano-7β-cyclopropyl-17α-methyl-19-nor-androst-4-en-3-on:

¹H-NMR (300 MHz, CDCl₃ TMS als interner Standard, ausgewählte Signale): δ = 0,12(m,1H), 0,30(m,1H), 0,59(m,4H), 0,87(m,1H), 1,12(s,3H), 1,30(s,3H), 5,81(s,1H, H-4)

### Beispiel 28

### 17β-Cyano-17α-methyl -15β,16β-methylen-19-nor-androsta-4,6-dien-3-on

Eine Suspension von 3,4 g 17β-Cyano-17α-methyl-15β,16β-methylen 3-methoxy-19-nor-androsta-3,5-dien in 100 ml 1-Methyl-2-pyrrolidon wurde nacheinander bei 0°C mit 4 ml einer 10%igen Natrumacetatlösung sowie bei dieser Temperatur mit 1,6 g 1,3-Dibrom-5,5-dimethylhydantoin portionsweise versetzt, 0,5 Stunden bei 0°C (Eisbad) gerührt, mit 1,5 g Lithiumbromid sowie 1,3 g Lithiumcarbonat versetzt, und 3,5 Stunden bei 100°C Badtemperatur gerührt. Anschließend wurde in ein Gemisch aus Eiswasser und Kochsalzlösung eingerührt, der Niederschlag abfiltriert und der Filterkuchen aus Dimethoxyethan umkristallisiert. Man erhielt 17β-Cyano-17α-methyl - 15β,16β-methylen-19-nor-androsta-4,6-dien-3-on.

### 17β-Cyano-17α-methyl-15β,16β-methylen-19-nor-androsta-4,6-dien-3-on

¹H-NMR (300 MHz, CDCl₃ TMS als interner Standard, ausgewählte Signale): δ = 0,55(m,1H, cyclopropyl) 1,18 (m,1H, cyclopropyl), 1,25(s,3H, 18-CH3), 1,44(s,3H, 17-CH3), 5,85(s,1H, H-4), 6,29(m,1H, H-6), 6,45(m,1H,H-7)

### Beispiel 29

### 17β-Cyano-17a-ethyl-15β,16β-methylen-19-nor-androst-4-en-3-on

### 29a.

### 17β-Cyano-17α-ethyl-15β,16β-methylen 3-methoxy-19-nor-androsta-3,5-dien

17β-Cyano-15β,16β-methylen 3-methoxy-19-nor-androsta-3,5-dien wurde, wie in Beispiel 10a beschrieben, umgesetzt. Statt des dort eingesetzten Methyliodids wurde hier mit Ethyliodid gearbeitet. Man erhielt 17β-Cyano-17α-ethyl-15β,16β-methylen 3-methoxy-19-nor-androsta-3,5-dien.

### 29b.

### 17β-Cyano-17α-ethyl-15β,16β-methylen-19-nor-androst-4-en-3-on

Die in Beispiel 19a beschriebene Verbindung wurde analog der in Beispiel 10b angegebenen Vorschrift umgesetzt. Man erhielt 17β-Cyano-17α-ethyl-15β,16β-methylen-19-nor-androst-4-en-3-on.

### 17β-Cyano-17α-ethyl-15β,16β-methylen-19-nor-androst-4-en-3-on:

¹H-NMR (CDCl3) : 0,46(m,1H, cyclopropyl) 0,87 (m,1H, cyclopropyl), 1,08 (m,1H, cyclopropyl), 1,21 (m,7H, 18-CH3,17-CH2-CH3, cyclopropyl), 5,86(s,1H, H-4)

### Beispiel 30

### 17β-Cyano-17α-ethyl-6β-hydroxymethyl-15β,16β-methylen-19-nor-androst-4-en-3-on

17β-Cyano-17α-ethyl-15β,16β-methylen-19-nor-androst-4-en-3-on wurde analog Beispiel 7 umgesetzt, und man erhielt 17β-Cyano-17α-ethyl-6β-hydroxymethyl-15β,16β-methylen-19-nor-androst-4-en-3-on.

### 17ß-Cyano-17α-ethyl-6ß-hydroxymethyl-15ß,16ß-methylen-19-nor-androst-4-en-3-on:

¹H-NMR (CDCl3) : 0,46(m,1H,cyclopropyl), 1,19(m,6H, 17-CH2-CH3, 18-CH3), 3,74(m,2H,CH2OH) 5,94(s,1H, H-4)

### Beispiel 31

### 17ß-Cyano-17α-ethyl-6,6-ethylen-15ß,16ß-methylen-19-nor-androst-4-en-3-on

17ß-Cyano-17α-ethyl-6ß-hydroxymethyl-15ß,16ß-methylen-19-nor-androst-4-en-3-on wurde analog Beispiel 8 umgesetzt, man erhielt 17ß-Cyano-17α-ethyl-6,6-ethylen-15ß,16ß-methylen-19-nor-androst-4-en-3-on.

### 17ß-Cyano-17α-ethyl-6,6-ethylen-15ß,16ß-methylen-19-nor-androst-4-en-3-on:

¹H-NMR (CDCl3) : 0,43(m,2H,6,6-ethylen/cyclopropyl), 0,59, 0,79, 0,96, 1,08(4 x m,4H,6,6-ethylen), 1,22(m,6H, 17-CH2-CH3, 18-CH3) 5,70(s,1H, H-4)

### Beispiel 32

### 17ß-Cyano-17α-ethyl-15ß,16ß-methylen-19-nor-androsta-4,6-dien-3-on

Aus 17ß-Cyano-17α-ethyl-15ß,16ß-methylen 3-methoxy-19-nor-androsta-3,5-dien erhielt man analog der in Beispiel 2b angegebenen Vorschrift 17ß-Cyano-17α-ethyl-15ß,16ß-methylen-19-nor-androsta-4,6-dien-3-on.

### 17ß-Cyano-17α-ethyl-15ß,16ß-methylen-19-nor-androsta-4,6-dien-3-on:

¹H-NMR (CDCl3) : 0,53(m,1H, cyclopropyl) 1,09-1,28 (m,9H, 18-CH3, 17-CH2-CH3 cyclopropyl), 5,80(s,1H, H-4), 6,25(m,1H, H-6), 6,40(m,1H,H-7)

### Beispiel 33

### 17ß-Cyano-17α-ethyl-7α-methyl-15ß,16ß-methylen-19-nor-androst-4-en-3-on und 17ß-Cyano-17α-ethyl-7ß-methyl 15ß,16ß-methylen-19-nor-androst-4-en-3-on

17ß-Cyano-17α-ethyl-15ß,16ß-methylen-19-nor-androsta-4,6-dien-3-on wurde analog Beispiel 3 umgesetzt, und man erhielt nach der Chromatographie als Fraktion I 17ß-Cyano-17α-ethyl-7α-methyl-15ß,16ß-methylen-19-nor-androst-4-en-3-on und als Fraktion II 17ß-Cyano-17α-ethyl-7ß-methyl 15ß,16ß-methylen-19-nor-androst-4-en-3-on.

### 17ß-Cyano-17α-ethyl-7α-methyl-15ß,16ß-methylen-19-nor-androst-4-en-3-on:

¹H-NMR (CDCl3) : 0,45(m,1H, cyclopropyl), 0,87(d,3H,J=7,34, 7-CH3), 1,23 (m,6H, 18-CH3, 17-CH2-CH3), 5,86(s,1H, H-4)

### 17ß-Cyano-17α-ethyl-7ß-methyl 15ß,16ß-methylen-19-nor-androst-4-en-3-on:

¹H-NMR (CDCl3) : 0,53(m,1H, cyclopropyl), 1,22 (m,9H, 7-CH3, 18-CH3, 17-CH2-CH3), 5,82(s,1H, H-4)

### Beispiel 34

### 17ß-Cyano-17α,7α-diethyl-15ß,16ß-methylen-19-nor-androst-4-en-3-on und 17ß-Cyano-17α,7ß-diethyl-15ß,16ß-methylen-19-nor-androst-4-en-3-on

Nach der Methode des Beispiels 3 mit Ethylmagnesiumbromid in Ether anstelle des Methylmagnesiumbromids erhielt man aus 17ß-Cyano-17α-ethyl-15ß,16ß-methylen-19-nor-androsta-4,6-dien-3-on nach der Chromatographie als Fraktion I 17ß-Cyano-17α,7α-diethyl-15ß,16ß-methylen-19-nor-androst-4-en-3-on und als Fraktion II 17β-Cyano-17α,7ß-diethyl-15ß,16ß-methylen-19-nor-androst-4-en-3-on

### 17ß-Cyano-17α,7α-diethyl-15ß,16ß-methylen-19-nor-androst-4-en-3-on:

¹H-NMR (CDCl3) : 0,46 (m,1H,cyclopropyl) 0,92 (t,3H,J=7,34, 7-CH2-CH3), 1,23(m,6H, 18-CH3, 17-CH2-CH3), 5,87(s,1H, H-4)

### 17ß-Cyano-17α,7β-diethyl-15ß,16ß-methylen-19-nor-androst-4-en-3-on:

¹H-NMR (CDCl3) : 0,54 (m,1H,cyclopropyl) 0,94 (t,3H,J=7,34, 7-CH2-CH3), 1,21(t,3H, J=7,34,17-CH2-CH3) 1,24(s,3H, 18-CH3), 5,84(s,1H, H-4)

### Beispiel 35

### 17ß-Cyano-17α-ethyl-7α-vinyl-15ß, 16ß-methylen-19-nor-androst-4-en-3-on und 17ß-Cyano-17α-ethyl-7ß-vinyl-15ß,16ß-methylen-19-nor-androst-4-en-3-on

Nach der Methode des Beispiels 3 mit Vinylmagnesiumbromid anstelle des Methylmagnesiumbromids erhält man aus 17ß-Cyano-17α-ethyl-15ß,16ß-methylen-19-nor-androsta-4,6-dien-3-on nach der Chromatographie als Fraktion I 17ß-Cyano-17α-ethyl-7α-vinyl-15ß,16ß-methylen-19-nor-androst-4-en-3-on und als Fraktion II 17β-Cyano-17α-ethyl-7ß-vinyl-15ß,16ß-methylen-19-nor-androst-4-en-3-on.

### 17ß-Cyano-17α-ethyl -7α-vinyl-15ß,16ß-methylen-19-nor-androst-4-en-3-on:

¹H-NMR (CDCl3) : 0.46 (m,1H,cyclopropyl), 1.08(m,1H,cyclopropyl) 1.22(m,3H, CH2-CH3),1.27(s,3H,18-CH3),5.17(m,2H, CH2=CH), 5.81 (m,1H,CH2=CH) 5.87(s,1H, H-4)

### 17ß-Cyano-17α-ethyl-7ß-vinyl-15ß,16ß-methylen-19-nor-androst-4-en-3-on:

¹H-NMR (CDCl3) : 0.42 (m,1H,cyclopropyl), 0.99(m,1H,cyclopropyl) 1.24(m,6H, 18-CH3, CH2-CH3),5.02(m,2H, CH2=CH), 5.85(s,1H, H-4), 5.90 (m,1H,CH2=CH)

### Beispiel 36

### 17ß-Cyano-17α-ethyl-7α-cyclopropyl-15ß,16ß-methylen-19-nor-androst-4-en-3-on und 17ß-Cyano-17α-ethyl-7ß-cyclopropyl-15ß,16ß-methylen-19-nor-androst-4-en-3-on

Nach der Methode des Beispiels 3 mit Cyclopropylmagnesiumbromid anstelle des Methylmagnesiumbromids erhält man aus 17ß-Cyano-17α-ethyl-15ß,16ß-methylen-19-nor-androsta-4,6-dien-3-on nach der Chromatographie als Fraktion I 17ß-Cyano-17α-ethyl-7α-cyclopropyl-15ß,16ß-methylen-19-nor-androst-4-en-3-on und als Fraktion II 17ß-Cyano-17α-ethyl-7ß-cyclopropyl-15ß,16ß-methylen-19-nor-androst-4-en-3-on.

### 17ß-Cyano-17α-ethyl-7α-cyclopropyl-15ß,16ß-methylen-19-nor-androst-4-en-3-on:

¹H-NMR (CDCl3) : -0.05(m,1H,cyclopropyl), 0.26(m,1H,cyclopropyl), 0.42(m,3H, cyclopropyl), 1.22(m,6H, CH2-CH3, 18-CH3), 5.90(s,1H, H-4)

### 17ß-Cyano-17α-ethyl-7ß-cyclopropyl-15ß,16ß-methylen-19-nor-androst-4-en-3-on:

¹H-NMR (CDCl3) : 0.25(m,1H,cyclopropyl), 0.33(m,1H,cyclopropyl), 0.47(m,1H,cyclopropyl), 0.60(m,2H, cyclopropyl), 1.06(m,1H, cyclopropyl), 1.22(m,3H, CH2-CH3),1.27(s,3H, 18-CH3), 5.81(s,1H, H-4)

### Beispiel 37

### 17ß-Cyano-17α-ethyl-6ß,7ß-methylen-15ß,16ß-methylen-19-nor-androst-4-en-3-on und 17ß-Cyano-17α-ethyl-6α,7α-methylen-15ß,16ß-methylen-19-nor-androst-4-en-3-on

17ß-Cyano-17α-ethyl-15ß,16ß-methylen-19-nor-androsta-4,6-dien-3-on wird nach der in Beispiel 9 angegebenen Methode umgesetzt und man erhält nach der Chromatographie als Fraktion I 17ß-Cyano-17α-ethyl-6ß,7ß-methylen-15ß,16ß-methylen-19-nor-androst-4-en-3-on und als Fraktion II 17ß-Cyano-17α-ethyl-6α,7α-methylen-15ß,16ß-methylen-19-nor-androst-4-en-3-on

### 17ß-Cyano-17α-ethyl-6ß,7ß-methylen-15ß,16ß-methylen-19-nor-androst-4-en-3-on:

¹H-NMR (CDCl₃): 0.49(m,1H,cyclopropyl), 0.78(m,2H,cyclopropyl), 0.96(m,1H,cyclopropyl), 1.13(m,1H, cyclopropyl), 1.23(m,6H, CH2-CH3 ,18-CH3),6.05(s,1H, H-4)

### 17ß-Cyano-17α-ethyl-6α,7α-methylen-15ß,16ß-methylen-19-nor-androst-4-en-3-on :

¹H-NMR (CDCl₃): 0.52(m,1H,cyclopropyl), 0.59(m,1H,cyclopropyl), 0.97(m,1H,cyclopropyl), 1.17(m,1H, cyclopropyl), 1.18(s,3H,18-CH3),1.23(m,3H, CH2-CH3,),6.12(s,1H, H-4)

### Beispiel 38

### 17ß-Cyano-17α,7α-dimethyl-15ß,16ß-methylen-19-nor-androst-4-en-3-on und 17ß-Cyano-17α,7ß-dimethyl 15ß,16ß-methylen-19-nor-androst-4-en-3-on

17ß-Cyano-17α-methyl -15ß,16ß-methylen-19-nor-androsta-4,6-dien-3-on wird analog Beispiel 3 umgesetzt und man erhält nach der Chromatographie als Fraktion I 17ß-Cyano-17α,7α-dimethyl-15ß,16ß-methylen-19-nor-androst-4-en-3-on und als Fraktion II 17ß-Cyano-17α,7ß-dimethyl 15ß,16ß-methylen-19-nor-androst-4-en-3-on.

### 17ß-Cyano-17α, 7α-dimethyl-15ß,16ß-methylen-19-nor-androst-4-en-3-on

¹H-NMR (CDCl3) : 0.44 (m,1H,cyclopropyl) 0.88 (d,3H,J=6.97Hz,7-CH3), 1.08(m,1H,cyclopropyl) 1.20(s,3H, 18-CH3), 1.40(s,3H, 17-CH3), 5.86(s,1H, H-4)

### 17ß-Cyano-17α,7ß-dimethyl-15ß,16ß-methylen-19-nor-androst-4-en-3-on .

¹H-NMR (CDCl3) : 0.51 (m,1H,cyclopropyl), 0.98 (m,1H,cyclopropyl), 1.06 (m,1H,cyclopropyl), 1.20(s,3H, 18-CH3), 1.22 (d,3H,J=5.87Hz,7-CH3), 1.38(s,3H,17-CH3), 5.83(s, 1H, H-4)

### Beispiel 39

### 17ß-Cyano-17α-methyl-7α-ethyl-15ß,16ß-methylen-19-nor-androst-4-en-3-on und 17ß-Cyano-17α-methyl-7ß-ethyl-15ß,16ß-methylen-19-nor-androst-4-en-3-on

17ß-Cyano-17α-methyl-15ß,16ß-methylen-19-nor-androsta-4,6-dien-3-on wird nach der Methode des Beispiels 3 mit Ethylmagnesiumbromid in Ether anstelle des Methylmagnesiumbromids umgesetzt und nach der Chromatographie erhielt man als Fraktion I 17ß-Cyano-17α-methyl-7α-ethyl-15ß,16ß-methylen-19-nor-androst-4-en-3-on und als Fraktion II 17ß-Cyano-17α-methyl-7ß-ethyl-15ß,16ß-methylen-19-nor-androst-4-en-3-on.

### 17ß-Cyano-17α-methyl-7α-ethyl-15ß,16ß-methylen-19-nor-androst-4-en-3-on:

¹H-NMR (CDCl3) : 0.45 (m,1H,cyclopropyl) 0.92 (m,3H, 7-CH3-CH2),1.20(s,3H, 18-CH3),1.39(s,3H,17-CH3), 5.87(s,1H, H-4)

### 17ß-Cyano-17α-methyl 7ß-ethyl-15ß,16-methylen-19-nor-androst-4-en-3-on:

¹H-NMR (CDCl3) : 0.52 (m,1H,cyclopropyl) 0.94 (m,3H, 7-CH2-CH3), 1.07(m,1H,cyclopropyl) 1.21(s,3H, 18-CH3),1.38(s,3H,17-CH3), 5.84(s,1H, H-4)

### Beispiel 40

### 17ß-Cyano-17α-methyl-7α-vinyl-15ß,16ß-methylen-19-nor-androst-4-en-3-on und 17ß-Cyano-17α-methyl-7ß-vinyl-15ß,16ß-methylen-19-nor-androst-4-en-3-on

17ß-Cyano-17α-methyl-15ß,16ß-methylen-19-nor-androsta-4,6-dien-3-on wird nach der Methode des Beispiels 3 mit Vinylmagnesiumbromid anstelle des Methylmagnesiumbromids umgesetzt und man erhält nach der Chromatographie als Fraktion I 17ß-Cyano-17α-methyl-7α-vinyl-15ß,16ß-methylen-19-nor-androst-4-en-3-on und als Fraktion II 17ß-Cyano-17α-methyl-7ß-vinyl-15ß,16ß-methylen-19-nor-androst-4-en-3-on.

### 17ß-Cyano-17ß-methyl -7α-vinyl-15ß,16ß-methylen-19-nor-androst-4-en-3-on:

¹H-NMR (CDCl3) : 0.45 (m,1H,cyclopropyl), 1.09(m,1H,cyclopropyl) 1.19(s,3H, 18-CH3), 1.37(s,3H, 17-CH3),5.16(m,2H, CH2=CH), 5.82 (m,1H,CH2=CH) 5.87(s,1H, H-4)

### 17ß-Cyano-17α-methyl -7ß-vinyl-15ß,16ß-methylen-19-nor-androst-4-en-3-on:

¹H-NMR (CDCl3) : 0.40 (m,1H,cyclopropyl), 0.98(m,2H,cyclopropyl) 1.20(s,3H, 18-CH3), 1.36(s,3H, 17-CH3),5.03(m,2H, CH2=CH), 5.85(s,1H, H-4), 5.90 (m,1H,CH2=CH)

### Beispiel 41

### 17ß-Cyano-17α-methyl-7α-cyclopropyl-15ß,16ß-methylen-19-nor-androst-4-en-3-on und 17ß-Cyano-17α-methyl-7ß-cyclopropyl-15ß,16ß-methylen-19-nor-androst-4-en-3-on

17ß-Cyano-17α-methyl-15ß,16ß-methylen-19-nor-androsta-4,6-dien-3-on wird nach der Methode des Beispiels 3 mit Cyclopropylmagnesiumbromid anstelle des Methylmagnesiumbromids umgesetzt und man erhält nach der Chromatographie als Fraktion I 17ß-Cyano-17α-methyl-7α-cyclopropyl-15ß, 16ß-methylen-19-nor-androst-4-en-3-on und als Fraktion II 17ß-Cyano-17α-methyl-7ß-cyclopropyl-15ß,16ß-methylen-19-nor-androst-4-en-3-on.

### 17ß-Cyano-17α-methyl-7α-cyclopropyl-15ß,16ß-methylen-19-nor-androst-4-en-3-on:

¹H-NMR (CDCl3) : 0.05(m,1H,cyclopropyl), 0.35(m,1H,cyclopropyl), 0.41(m,1H,cyclopropyl), 0.49(m,1H,cyclopropyl), 0.59(m,2H, cyclopropyl), 1.19(s,3H, 18-CH3), 1.41(s,3H, 17-CH3), 5.90(s,1H, H-4)

### 17ß-Cyano-17α-methyl-7ß-cyclopropyl-15ß,16ß-methylen-19-nor-androst-4-en-3-on:

¹H-NMR (CDCl3) : 0.25(m,1H,cyclopropyl), 0.33(m,1H,cyclopropyl), 0.45(m,1H,cyclopropyl), 0.60(m,2H, cyclopropyl), 0.79(m,1H,cyclopropyl), 0.87(m,1H, cyclopropyl), 0.94(m,1H, cyclopropyl), 1.07(m,1H, cyclopropyl), 1.22(s,3H, 18-CH3), 1.39(s,3H, 17-CH3), 5.82(s,1H, H-4)

### Beispiel 42

### 17ß-Cyano-17α-methyl-6ß-hydroxymethyl-15ß,16ß-methylen-19-nor-androst-4-en-3-on

17ß-Cyano-17α-methyl-15ß,16ß-methylen-19-nor-androst-4-en-3-on wird analog des in Beispiel 7 angegebenen Verfahrens umgesetzt. Man erhält 17ß-Cyano-17α-methyl-6ß-hydroxymethyl-15ß,16ß-methylen-19-nor-androst-4-en-3-on.

### 17ß-Cyano-17α-methyl-6ß-hydroxymethyl-15ß,16ß-methylen-19-nor-androst-4-en-3-on:

¹H-NMR (CDCl3) : 0.45(m,1H,cyclopropyl), 1.08(m,1H,cyclopropyl), 1.18(s,3H, 18-CH3), 1.38(s,3H, 17-CH3),3.74(m,2H,CH2OH) 5.94(s,1H, H-4)

### Beispiel 43

### 17ß-Cyano-17α-methyl-6,6-ethylen-15ß,16ß-methylen-19-nor-androst-4-en-3-on

17ß-Cyano-17ß-methyl-6ß-hydroxymethyl-15ß,16ß-methylen-19-nor-androst-4-en-3-on wird analog derm in den Beispielen 8a und 8b angegebenen Verfahren umgesetzt. Man erhält 17ß-Cyano-17α-methyl-6,6-ethylen-15ß,16ß-methylen-19-nor-androst-4-en-3-on.

### 17ß-Cyano-17α-methyl-6,6-ethylen-15ß,16ß-methylen-19-nor-androst-4-en-3-on:

¹H-NMR (CDCl3) : 0.42-1.08(m,6H,6,6-ethylen/cyclopropyl) 1.22(s,3H,18-CH3), 1.39(s,3H,17-CH3), 5.70(s,1H, H-4)

### Beispiel 44

### 17ß-Cyano-17α-methyl-6ß,7ß-methylen-15ß,16ß-methylen-19-nor-androst-4-en-3-on und 17ß-Cyano-17α-methyl-6α,7α-methylen-15ß,16ß-methylen-19-nor-androst-4-en-3-on

17ß-Cyano-17α-methyl-15ß,16ß-methylen-19-nor-androsta-4,6-dien-3-on wird nach der in Beispiel 9 angegebenen Methode umgesetzt und man erhält als Fraktion I 17ß-Cyano-17α-methyl-6ß,7ß-methylen-15ß,16ß-methylen-19-nor-androst-4-en-3-on und als Fraktion II 17ß-Cyano-17α-methyl-6α,7α-methylen-15ß,16ß-methylen-19-nor-androst-4-en-3-on

### 17ß-Cyano-17α-methyl-6ß,7ß-methylen-15ß,16ß-methylen-19-nor-androst-4-en-3-on:

¹H-NMR (CDCl₃): 0.47(m,1H,cyclopropyl), 0.80(m,2H,cyclopropyl), 0.97(m,1H,cyclopropyl), 1.13(m,1H, cyclopropyl),1.22(s,3H, 18-CH3), 1.40(s,3H, 17-CH3), 6.05(s,1H, H-4)

### 17ß-Cyano-17α-methyl -6α,7α-methylen-15ß,16ß-methylen-19-nor-androst-4-en-3-on:

¹H-NMR (CDCl₃): 0.50(m,1H,cyclopropyl), 0.59(m,1H,cyclopropyl), 0.98(m,1H,cyclopropyl), 1.16(s,3H, 18-CH3), 1.41 (s,3H, 17-CH3), 6.12(s,1H, H-4)

### Beispiel 45

### 17ß-Cyano-17α-methyl-6β, 7β-methylen-19-nor-androst-4-en-3-on und 17ß-Cyano-17α-methyl-6α, 7α-methylen-19-nor-androst-4-en-3-on

17ß-Cyano-17α-methyl-19-nor-androsta-4,6-dien-3-on wird nach der in Beispiel 9 angegebenen Methode umgesetzt und man erhält nach der Chromatographie als Fraktion I 17ß-Cyano-17α-methyl-6α, 7α-methylen-19-nor-androst-4-en-3-on und als Fraktion II 17ß-Cyano-17α-methyl-6β, 7β-methylen-19-nor-androst-4-en-3-on.

### 17ß-Cyano-17α-methyl-6α, 7α-methylen-19-nor-androst-4-en-3-on:

¹H-NMR (CDCl₃ ): 0.68 (m, 1H), 0.77 (m, 1H), 0.90 (m, 1H), 1.12 (s, 3H, CH₃), 1.32 (s, 3H, CH₃), 1.68 (m, 1H), 2.02 (m, 1H), 2,17 (m, 1H), 2.40 (m, 1H), 2.51 (m, 1H), 6.03 (s,1H, H-4)

### 17ß-Cyano-17α-methyl-6β, 7β-methylen-19-nor-androst-4-en-3-on:

¹H-NMR (CDCl₃ ): 0.52(m,1H), 0.93(m, 1H), 1.08 (s, 3H, CH₃), 1.33 (s, 3H, CH₃), 1.95 (m, 1H), 2.37-2.48 (m, 2H), 6.11 (s, 1H, H-4)

### Beispiel 46

### 4-Chlor-17ß-cyano-17α-ethyl-19-nor-androst-4-en-3-on

100 mg 17ß-Cyano-17α-ethyl-19-nor-androst-4-en-3-on werden in 1,1 ml Pyridin gelöst und auf 0°C gekühlt. Nach Zusatz von 42 µl Sulfurylchlorid wird 1,5 Stunden bei dieser Temperatur nachgerührt.

Nach Versetzten mit gesättigter wässriger Natriumhydrogencarbonatlösung, Wasser und Ethylacetat werden die Phasen getrennt und die organische Phase mit Wasser und gesättigter wässsriger Natriumchloridlösung gewaschen. Nach Trocknung der organischen Phase über Natriumsulfat und Filtration wird eingeengt und der Rückstand an Kieselgel mit einem Gemisch aus Ethylacetat und n-Hexan chromatographiert. Man erhält 4-Chlor-17ß-cyano-17α-ethyl-19-nor-androst-4-en-3-on.

### 4-Chlor-17ß-cyano-17α-ethyl-19-nor-androst-4-en-3-on:

¹H-NMR (d6-DMSO): 0.97 (t, 3H, J=7.3, -CH₂-CH₃), 1.00 (s, 3H, - CH₃), 1.99 (m, 1H), 2.08-2.22 (m, 2H), 3.10 (m, 1H)

### Beispiel 47

### 17ß-Cyano-3-hydroxyimino-17α-ethyl-19-nor-androst-4-en-3-on

100 mg 17ß-Cyano-17α-ethyl-19-nor-androst-4-en-3-on werden in 1 ml Pyridin gelöst und mit 34,5 mg Hydroxylaminhydrochlorid versetzt. Nach einstündigem Rühren bei 125°C Badtemperatur wird der Ansatz zwischen Wasser und Ethylacetat verteilt. Die organische Phase wird mit Wasser und gesättigter wässriger Natriumchloridlösung gewaschen, über Natriumsulfat getrocknet, filtriert und eingeengt. Nach Chromatographie an Kieselgel mit einem Gemisch aus Ethylacetat und n-Hexan wird das produkthaltige eluat eingeengt und aus einem Gemisch von Aceton und Diisopropylether umkristallisiert. Es wird 17ß-Cyano-3-hydroxyimino-17α-ethyl-19-nor-androst-4-en-3-on als E/Z-Gemisch der Oxime erhalten.

### 17ß-Cyano-3-hydroxyimino-17α-ethyl-19-nor-androst-4-en-3-on:

¹H-NMR (d6-DMSO): 0.41 (m, 1H), 0.96 (t, 3H, J=7.3, -CH₂-CH₃), 0.99 (s, 3H, -CH₃), 2.82 und 2.98 (jeweils m, zusammen 1H), 5.76 und 6.36 (jeweils s, zusammen 1H, H-4)

### Beispiel 48

### 17ß-Cyano-19-nor-androsta-4,9-dien-3-on

### 48a.

### 17ß-Cyano-3,3-dimethoxyestr-5(10)-en

75 g 3,3-Dimethoxyestr-5(10)-en-17-on warden analog der in Beispiel 1d angegebenen Methode umgesetzt. Das so erhaltene Rohprodukt wird in einem Gemisch aus Diisopropylether und Hexan aufgenommen, vom Rückstand abfiltriert und das Filtrat eingeengt. Nach Kristallisation des Eindampfrückstandes aus Diisopropylether erhält man 17ß-Cyano-3,3-dimethoxyestr-5(10)-en

### 17ß-Cyano-3,3-dimethoxvestr-5(10)-en:

¹H-NMR (d6-DMSO): 0.84 (s, 3H, 17-CH₃), 1.46 (m, 1H), 1.70 (m, 1H), 2.57 (m, 1H), 3.07 (s, 3H, 3-OCH₃), 3.10 (s, 3H, 3-OCH₃)

### 48b.

### 17ß-Cyanoestr-5(10)-en-3-on

3 g 17ß-Cyano-3,3-dimethoxyestr-5(10)-en werden in einem Gemisch aus 24 ml Dichlormethan und 70 ml t-Butanol suspendiert. Nach Zusatz von 28 ml Wasser und 0,11 ml 60%-iger Perchlorsäure wird bis zur vollständigen Umsetzung gerührt, mit gesättigter wässriger Natriumhydrogencarbonat versetzt und mit Ethylacetat extrahiert. Nach Wäsche der organischen Phase mit gesättigter wässriger Natriumchloridlösung, Trocknung der organischen Phase über Natriumsulfat und Filtration wird als Eindampfrückstand des Filtrates 17ß-Cyanoestr-5(10)-en-3-on erhalten, welches ohne Reinigung weiterverarbeitet wird.

### 48c.

### 17ß-Cyano-19-nor-androsta-4,9-dien-3-on

2,4 g 17ß-Cyanoestr-5(10)-en-3-on werden mit 35 ml Pyridin und 3,2 g Pyridiniumhydrobromid-Perbromid versetzt. Es wird 1 Stunde bei Raumtemperatur und anschließend 4 Stunden bei 50°C gerührt. Nach Abkühlung wird mit 40 ml eiskalter 6n wässriger Salzsäure versetzt und mit Ethylacetat extrahiert. Nach Waschen der organischen Phase mit 1 n wässriger Salzsäure, gesättigter wässriger Natriumhyrogencarbonatlösung, Trocknung über Natriumsulfat und Filtration wird eingeengt und der Eindampfsrückstand mittels Chromatographie an Kieselgel mit einem Gemisch aus Ethylacetat und n-Hexan gereinigt. Man erhält 17ß-Cyano-19-nor-androsta-4,9-dien-3-on

### 17ß-Cyano-19-nor-androsta-4,9-dien-3-on:

¹H-NMR (d6-DMSO): 0.94 (s, 3H, 17-CH₃), 1.09-1.22 (m, 2H), 1.25-1.41 (m, 2H), 1.69 (m, 1H), 2.59 (m, 1H), 2.75-2.90 (m, 2H), 5,56 (s, 1H, H-4)

## Patentansprüche

1. 17ß-Cyano-19-nor-androst-4-en-Derivat mit der allgemeinen chemischen Formel (1) 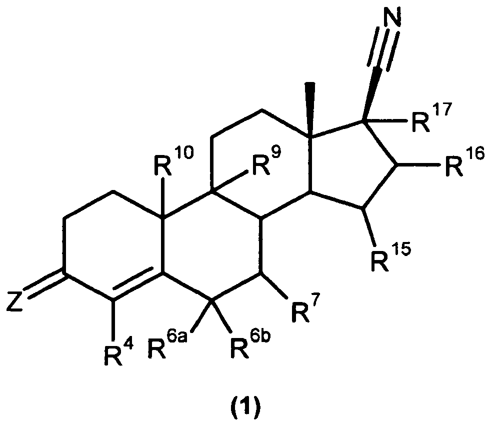 , namentlich
• 17ß-Cyano-6ß-hydroxymethylen-19-nor-androst-4-en-3-on,
• 17β-Cyano-17α-methyl-19-nor-androst-4-en-3-on,
• 17α-Allyl-17ß-cyano-19-nor-androst-4-en-3-on,
• 17ß-Cyano-17α-ethyl-19-nor-androst-4-en-3-on,
• 17ß-Cyano-6,6-ethandiyl-19-nor-androst-4-en-3-on,
• 17ß-Cyano-6ß,7ß-methylen-19-nor-androst-4-en-3-on,
• 17ß-Cyano-6α,7α-methylen-19-nor-androst-4-en-3-on,
• 17ß-Cyano-17α-methyl-6ß-hydroxymethylen-19-nor-androst-4-en-3-on,
• 17ß-Cyano-15ß,16ß-methylen-19-nor-androst-4-en-3-on,
• 17ß-Cyano-6,6-ethandiyl-17α-methyl-19-nor-androst-4-en-3-on,
• 17ß-Cyano-7α-ethyl-19-nor-androst-4-en-3-on,
• 17ß-Cyano-17α-methyl-6α,7α-methylen-19-nor-androst-4-en-3-on,
• 17ß-Cyano-17α-methyl-6ß,7ß-methylen-19-nor-androst-4-en-3-on,
• 17ß-Cyano-7ß-ethyl-19-nor-androst-4-en-3-on,
• 17ß-Cyano-19-nor-androsta-4,6-dien-3-on,
• 17ß-Cyano-17α-methyl-19-nor-androsta-4,6-dien-3-on,
• 17ß-Cyano-17α-methyl-15ß, 16ß-methylen-19-nor-androst-4-en-3-on,
• 17ß-Cyano-17α-ethyl-15ß, 16ß-methylen-19-nor-androst-4-en-3-on,
• 7α,17α-Bismethyl-17ß-cyano -19-nor-androst-4-en-3-on,
• 17ß-Cyano-7α-methyl-19-nor-androst-4-en-3-on,
• 17ß-Cyano-7ß-methyl-19-nor-androst-4-en-3-on,
• 17ß-Cyano-7α-vinyl-19-nor-androst-4-en-3-on,
• 17ß-Cyano-7ß-vinyl-19-nor-androst-4-en-3-on,
• 17ß-Cyano-7α-cyclopropyl-19-nor-androst-4-en-3-on,
• 17ß-Cyano-7ß-cyclopropyl-19-nor-androst-4-en-3-on,
• 17ß-Cyano-7α-cyclopropyl-17α-methyl-19-nor-androst-4-en-3-on,
• 17ß-Cyano-7ß-cyclopropyl-17α-methyl-19-nor-androst-4-en-3-on,
• 17ß-Cyano-17α-methyl-7α-vinyl-19-nor-androst-4-en-3-on,
• 17ß-Cyano-17α-methyl-7ß-vinyl-19-nor-androst-4-en-3-on,
• 17ß-Cyano-15ß,16ß-methylen-19-nor-androsta-4,6-dien-3-on,
• 17ß-Cyano-15ß,16ß-methylen-6ß-hydroxymethylen-19-nor-androst-4-en-3-on,
• 17α-Ethyl-17ß-cyano-15ß,16ß-methylen-6ß-hydroxymethyl-19-nor-androst-4-en-3-on,
• 17ß-Cyano-6ß, 7ß-15ß,16ß-bismethylen-19-nor-androst-4-en-3-on,
• 17ß-Cyano-6α, 7α-15α, 16ß-bismethylen-19-nor-androst-4-en-3-on,
• 17ß-Cyano-7ß-cyclopropyl-15ß,16ß-methylen-19-nor-androst-4-en-3-on,
• 17ß-Cyano-7α-cyclopropyl-15ß,16ß-methylen-19-nor-androst-4-en-3-on,
• 17ß-Cyano-7ß-ethyl-15ß, 16ß-methylen-19-nor-androst-4-en-3-on,
• 17ß-Cyano-7α-ethyl-15ß, 16ß-methylen-19-nor-androst-4-en-3-on,
• 17ß-Cyano-7ß-methyl-15ß,16ß-methylen-19-nor-androst-4-en-3-on,
• 17ß-Cyano-7α-methyl-15ß,16ß-methylen-19-nor-androst-4-en-3-on,
• 17ß-Cyano-17α-methyl-15ß,16ß-methylen-19-nor-androsta-4,6-dien-3-on,
• 17ß-Cyano-17α-ethyl-15ß,16ß-methylen-19-nor-androsta-4,6-dien-3-on,
• 17ß-Cyano-15ß,16ß-methylen-7ß-vinyl-19-nor-androst-4-en-3-on,
• 17ß-Cyano-15ß,16ß-methylen-7α-vinyl-19-nor-androst-4-en-3-on,
• 17ß-Cyano-6,6-ethandiyl-15ß,16ß-methylen-19-nor-androst-4-en-3-on,
• 17ß-Cyano-15α,16α-methylen-19-nor-androst-4-en-3-on,
• 17ß-Cyano-17α,7α-dimethyl-15ß,16ß-methylen-19-nor-androst-4-en-3-on,
• 17ß-Cyano-17α,7ß-dimethyl-15ß,16ß-methylen-19-nor-androst-4-en-3-on,
• 17ß-Cyano-17α-methyl-7α-ethyl-15ß,16ß-methylen-19-nor-androst-4-en-3-on,
• 17ß-Cyano-17α-methyl 7ß-ethyl-15ß,16ß-methylen-19-nor-androst-4-en-3-on,
• 17ß-Cyano-17α-methyl -7α-vinyl-15ß,16ß-methylen-19-nor-androst-4-en-3-on,
• 17ß-Cyano-17α-methyl -7ß-vinyl-15ß,16ß-methylen-19-nor-androst-4-en-3-on,
• 17ß-Cyano-17α-methyl-7α-cyclopropyl-15ß, 16ß-methylen-19-nor-androst-4-en-3-on,
• 17ß-Cyano-17α-methyl-7ß-cyclopropyl-15ß,16ß-methylen-19-nor-androst-4-en-3-on,
• 17ß-Cyano-17α-methyl-6ß-hydroxymethyl-15ß, 16ß-methylen-19-nor-androst-4-en-3-on,
• 17ß-Cyano-17α-methyl-6,6-ethylen-15ß, 16ß-methylen-19-nor-androst-4-en-3-on,
• 17ß-Cyano-17α-methyl-6ß,7ß-methylen-15ß,16ß-methylen-19-nor-androst-4-en-3-on,
• 17ß-Cyano-17α-methyl-6α,7α-methylen-15ß, 16ß-methylen-19-nor-androst-4-en-3-on,
• 17ß-Cyano-17α-ethyl-7α-methyl-15ß,16ß-methylen-19-nor-androst-4-en-3-on,
• 17ß-Cyano-17α-ethyl-7ß-methyl 15ß,16ß-methylen-19-nor-androst-4-en-3-on,
• 17ß-Cyano-17α,7α-diethyl-15ß,16ß-methylen-19-nor-androst-4-en-3-on,
• 17ß-Cyano-17α,7ß-diethyl-15ß,16ß-methylen-19-nor-androst-4-en-3-on,
• 17ß-Cyano-17α-ethyl -7α-vinyl-15ß,16ß-methylen-19-nor-androst-4-en-3-on,
• 17ß-Cyano-17α-ethyl-7ß-vinyl-15ß,16ß-methylen-19-nor-androst-4-en-3-on,
• 17ß-Cyano-17α-ethyl-7α-cyclopropyl-15ß,16ß-methylen-19-nor-androst-4-en-3-on,
• 17ß-Cyano-17α-ethyl-7ß-cyclopropyl-15ß,16ß-methylen-19-nor-androst-4-en-3-on,
• 17ß-Cyano-17α-ethyl-6,6-ethylen-15ß,16ß-methylen-19-nor-androst-4-en-3-on,
• 17ß-Cyano-17α-ethyl-6ß,7ß-methylen-15ß, 16ß-methylen-19-nor-androst-4-en-3-on oder
• 17ß-Cyano-17α-ethyl-6α,7α-methylen-15ß,16ß-methylen-19-nor-androst-4-en-3-on.

2. Verwendung des 17ß-Cyano-19-nor-androst-4-en-Derivats nach dem Anspruch 1 zur Herstellung eines Arzneimittels zur oralen Kontrazeption und zur Behandlung von prä-, peri- und postmenopausalen Beschwerden.

3. Verwendung nach Anspruch 2, **dadurch gekennzeichnet, dass** das Arzneimittel gestagene und antimineralcorticoide Wirkung aufweist.

4. Arzneimittel enthaltend mindestens ein 17ß-Cyano-19-nor-androst-4-en-Derivat nach Anspruch 1 sowie mindestens einen geeigneten pharmazeutisch unbedenklichen Zusatzstoff.

5. Arzneimittel nach Anspruch 4, enthaltend außerdem mindestens ein Estrogen.

6. Arzneimittel nach Anspruch 5, **dadurch gekennzeichnet, dass** das Estrogen Ethinylestradiol ist.

7. Arzneimittel nach Anspruch 5, **dadurch gekennzeichnet, dass** das Estrogen ein natürliches Estrogen ist.

8. Arzneimittel nach Anspruch 7, **dadurch gekennzeichnet, dass** das natürliche Estrogen Estradiol ist.

9. Arzneimittel nach Anspruch 7, **dadurch gekennzeichnet, dass** das natürliche Estrogen Estradiolvalerat ist.

10. Arzneimittel nach Anspruch 7, **dadurch gekennzeichnet, dass** das natürliche Estrogen ein konjugiertes Estrogen ist.

## Claims

1. 17ß-Cyano-19-nor-androst-4-ene derivative having the general chemical formula (1) 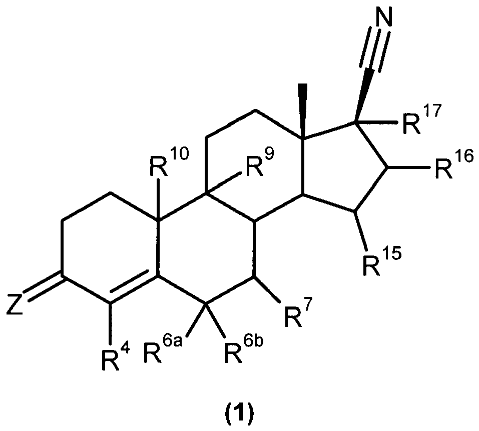 namely
• 17ß-cyano-6ß-hydroxymethylene-19-nor-androst-4-en-3-one,
• 17β-cyano-17α-methyl-19-nor-androst-4-en-3-one,
• 17α-Allyl-17ß-cyano-19-nor-androst-4-en-3-one,
• 17ß-cyano-17α-ethyl-19-nor-androst-4-en-3-one,
• 17ß-cyano-6,6-ethanediyl-19-nor-androst-4-en-3-one,
• 17ß-cyano-6ß,7ß-methylene-19-nor-androst-4-en-3-one,
• 17ß-cyano-6α,7α-methylene-19-nor-androst-4-en-3-one,
• 17ß-cyano-17α-methyl-6ß-hydroxymethylene-19-nor-androst-4-en-3-one,
• 17ß-cyano-15ß,16ß-methylene-19-nor-androst-4-en-3-one,
• 17ß-cyano-6,6-ethanediyl-17α-methyl-19-nor-androst-4-en-3-one,
• 17ß-cyano-7α-ethyl-19-nor-androst-4-en-3-one,
• 17ß-cyano-17α-methyl-6α,7α-methylene-19-nor-androst-4-en-3-one,
• 17ß-cyano-17α-methyl-6ß,7ß-methylene-19-nor-androst-4-en-3-one,
• 17ß-cyano-7ß-ethyl-19-nor-androst-4-en-3-one,
• 17ß-cyano-19-nor-androst-4,6-dien-3-one,
• 17ß-cyano-17α-methyl-19-nor-androst-4,6-dien-3-one,
• 17ß-cyano-17α-methyl-15ß,16ß-methylene-19-nor-androst-4-en-3-one,
• 17ß-cyano-17α-ethyl-15ß,16ß-methylene-19-nor-androst-4-en-3-one,
• 7α,17α-bismethyl-17ß-cyano-19-nor-androst-4-en-3-one,
• 17ß-cyano-7α-methyl-19-nor-androst-4-en-3-one,
• 17ß-cyano-7ß-methyl-19-nor-androst-4-en-3-one,
• 17ß-cyano-7α-vinyl-19-nor-androst-4-en-3-one,
• 17ß-cyano-7ß-vinyl-19-nor-androst-4-en-3-one,
• 17ß-cyano-7α-cyclopropyl-19-nor-androst-4-en-3-one,
• 17ß-cyano-7ß-cyclopropyl-19-nor-androst-4-en-3-one,
• 17ß-cyano-7α-cyclopropyl-17α-methyl-19-nor-androst-4-en-3-one,
• 17ß-cyano-7ß-cyclopropyl-17α-methyl-19-nor-and rost-4-en-3-one,
• 17ß-cyano-17α-methyl-7α-vinyl-19-nor-androst-4-en-3-one,
• 17ß-cyano-17α-methyl-7ß-vinyl-19-nor-androst-4-en-3-one,
• 17ß-cyano-15ß,16ß-methylene-19-nor-androst-4,6-dien-3-one,
• 17ß-cyano-15ß,16ß-methylene-6ß-hydroxymethylene-19-nor-androst-4-en-3-one,
• 17α -ethyl-17ß-cyano-15ß,16ß-methylene-6ß-hydroxymethyl-19-nor-androst-4-en-3-one,
• 17ß-cyano-6ß, 7ß-15ß,16ß-bismethylene-19-nor-androst-4-en-3-one,
• 17ß-cyano-6α,7α-15ß,16ß-bismethylene-19-nor-androst-4-en-3-one,
• 17ß-cyano-7ß-cyclopropyl-15ß,16ß-methylene-19-nor-androst-4-en-3-one,
• 17ß-cyano-7α-cyclopropyl-15ß,16ß-methylene-19-nor-androst-4-en-3-one,
• 17ß-cyano-7ß-ethyl-15ß,16ß-methylene-19-nor-androst-4-en-3-one,
• 17ß-cyano-7α-ethyl-15ß,16ß-methylene-19-nor-androst-4-en-3-one,
• 17ß-cyano-7ß-methyl-15ß,16ß-methylene-19-nor-androst-4-en-3-one,
• 17ß-cyano-7α-methyl-15ß,16ß-methylene-19-nor-androst-4-en-3-one,
• 17ß-cyano-17α-methyl-15ß,16ß-methylene-19-nor-androst-4,6-dien-3-one,
• 17ß-cyano-17α-ethyl-15ß,16ß-methylene-19-nor-androst-4,6-dien-3-one,
• 17ß-cyano-15ß,16ß-methylene-7ß-vinyl-19-nor-androst-4-en-3-one,
• 17ß-cyano-15ß,16ß-methylene-7α-vinyl-19-nor-androst-4-en-3-one,
• 17ß-cyano-6,6-ethanediyl-15ß,16ß-methylene-19-nor-androst-4-en-3-one,
• 17ß-cyano-15α,16α-methylene-19-nor-androst-4-en-3-one,
• 17ß-cyano-17α,7α-dimethyl-15ß,16ß-methylene-19-nor-androst-4-en-3-one,
• 17ß-cyano-17α,7ß-dimethyl-15ß,16ß-methylene-19-nor-androst-4-en-3-one,
• 17ß-cyano-17α-methyl-7α-ethyl-15ß,16ß-methylene-19-nor-androst-4-en-3-one,
• 17ß-cyano-17α-methyl7ß-ethyl-15ß,16ß-methylene-19-nor-androst-4-en-3-one,
• 17ß-cyano-17α-methyl-7α-vinyl-15ß,16ß-methylene-19-nor-androst-4-en-3-one,
• 17ß-cyano-17α-methyl-7ß-vinyl-15ß,16ß-methylene-19-nor-androst-4-en-3-one,
• 17ß-cyano-17α-methyl-7α-cyclopropyl-15ß,16ß-methylene-19-nor-androst-4-en-3-one,
• 17ß-cyano-17α-methyl-7ß-cyclopropyl-15ß,16ß-methylene-19-nor-androst-4-en-3-one,
• 17ß-cyano-17α-methyl-6ß-hydroxymethyl-15ß,16ß-methylene-19-nor-androst-4-en-3-one,
• 17ß-cyano-17α-methyl-6,6-ethylene-15ß,16ß-methylene-19-nor-androst-4-en-3-one,
• 17ß-cyano-17α-methyl-6ß,7ß-methylene-15ß,16ß-methylene-19-nor-androst-4-en-3-one,
• 17ß-cyano-17α-methyl-6α,7α-methylene-15ß,16ß-methylene-19-nor-androst-4-en-3-one,
• 17ß-cyano-17α-ethyl-7α-methyl-15ßl,16ß-methylene-19-nor-androst-4-en-3-one,
• 17ß-cyano-17α-ethyl-7ß-methyl15ß,16ß-methylene-19-nor-androst-4-en-3-one,
• 17ß-cyano-17α,7α-diethyl-15ß,16ß-methylene-19-nor-androst-4-en-3-one,
• 17ß-cyano-17α,7ß-diethyl-15ß,16ß-methylene-19-nor-androst-4-en-3-one,
• 17ß-cyano-17α-ethyl-7α-vinyl-15ß,16ß-methylene-19-nor-androst-4-en-3-one,
• 17ß-cyano-17α-ethyl-7ß-vinyl-15ß,16ß-methylene-19-nor-androst-4-en-3-one,
• 17ß-cyano-17α-ethyl-7α-cyclopropyl-15ß,16ß-methylene-19-nor-androst-4-en-3-one,
• 17ß-cyano-17α-ethyl-7ß-cyclopropyl-15ß,16ß-methylene-19-nor-androst-4-en-3-one,
• 17ß-cyano-17α-ethyl-6,6-ethylene-15ß,16ß-methylene-19-nor-androst-4-en-3-one,
• 17ß-cyano-17α-ethyl-6ß,7ß-methylene-15ß,16ß-methylene-19-nor-androst-4-en-3-one, or
• 17ß-cyano-17α-ethyl-6α,7α-methylene-15ß,16ß-methylene-19-nor-androst-4-en-3-one.

2. Use of the 17ß-cyano-19-nor-androst-4-ene derivative according to Claim 1 for the production of a medicament for oral contraception and for the treatment of pre-, peri- and postmenopausal symptoms.

3. Use according to Claim 2, **characterized in that** the medicament has gestagenic and antimineralcorticoid action.

4. Medicament comprising at least one 17ß-cyano-19-nor-androst-4-ene derivative according to Claim 1 and at least one suitable pharmaceutically harmless additive.

5. Medicament according to Claim 4, moreover comprising at least one oestrogen.

6. Medicament according to Claim 5, **characterized in that** the oestrogen is ethinylestradiol.

7. Medicament according to Claim 5, **characterized in that** the oestrogen is a natural oestrogen.

8. Medicament according to Claim 7, **characterized in that** the natural oestrogen is oestradiol.

9. Medicament according to Claim 7, **characterized in that** the natural oestrogen is oestradiol valerate.

10. Medicament according to Claim 7, **characterized in that** the natural oestrogen is a conjugated oestrogen.

## Revendications

1. Dérivé de 17β-cyano-19-nor-androst-4-ène correspondant à la formule chimique générale (1) 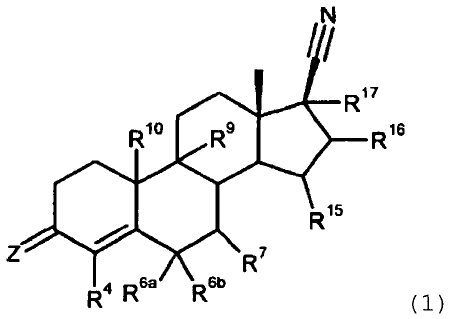 à savoir,
• 17β-cyano-6β-hydroxyméthylène-19-nor-androst-4-én-3-one,
• 17β-cyano-17α-méthyl-19-nor-androst-4-én-3-one,
• 17α-allyl-17β-cyano-19-nor-androst-4-én-3-one,
• 17β-cyano-17α-éthyl-19-nor-androst-4-én-3-one,
• 17β-cyano-6,6-éthanediyl-19-nor-androst-4-én-3-one,
• 17β-cyano-6β,7β-méthylène-19-nor-androst-4-én-3-one,
• 17β-cyano-6α,7α-méthylène-19-nor-androst-4-én-3-one,
• 17β-cyano-17α-méthyl-6β-hydroxyméthylène-19-nor-androst-4-én-3-one,
• 17β-cyano-15β,16β-méthylène-19-nor-androst-4-én-3-one,
• 17β-cyano-6,6-éthanediyl-17α-méthyl-19-nor-androst-4-én-3-one,
• 17β-cyano-7α-éthyl-19-nor-androst-4-én-3-one,
• 17β-cyano-17α-méthyl-6α,7α-méthylène-19-nor-androst-4-én-3-one,
• 17β-cyano-17α-méthyl-6β,7β-méthylène-19-nor-androst-4-én-3-one,
• 17β-cyano-7β-éthyl-19-nor-androst-4-én-3-one,
• 17β-cyano-19-nor-androsta-4,6-dién-3-one,
• 17β-cyano-17α-méthyl-19-nor-androsta-4,6-dién-3-one,
• 17β-cyano-17α-méthyl-15β,16β-méthylène-19-nor-androst-4-én-3-one,
• 17β-cyano-17α-éthyl-15β,16β-méthylène-19-nor-androst-4-én-3-one,
• 7α,17α-bisméthyl-17β-cyano-19-nor-androst-4-én-3-one,
• 17β-cyano-7α-méthyl-19-nor-androst-4-én-3-one,
• 17β-cyano-7β-méthyl-19-nor-androst-4-én-3-one,
• 17β-cyano-7α-vinyl-19-nor-androst-4-én-3-one,
• 17β-cyano-7β-vinyl-19-nor-androst-4-én-3-one,
• 17β-cyano-7α-cyclopropyl-19-nor-androst-4-én-3-one,
• 17β-cyano-7β-cyclopropyl-19-nor-androst-4-én-3-one,
• 17β-cyano-7α-cyclopropyl-17α-méthyl-19-nor-androst-4-én-3-one,
• 17β-cyano-7β-cyclopropyl-17α-méthyl-19-nor-androst-4-én-3-one,
• 17β-cyano-17α-méthyl-7α-vinyl-19-nor-androst-4-én-3-one,
• 17β-cyano-17α-méthyl-7β-vinyl-19-nor-androst-4-én-3-one,
• 17β-cyano-15β,16β-méthylène-19-nor-androsta-4,6-dién-3-one,
• 17β-cyano-15β,16β-méthylène-6β-hydroxyméthylène-19-nor-androst-4-én-3-one,
• 17α-éthyl-17β-cyano-15β,16β-méthylène-6β-hydroxyméthyl-19-norandrost-4-én-3-one,
• 17β-cyano-6β,7β-15β,16β-bisméthylène-19-nor-androst-4-én-3-one,
• 17β-cyano-6α,7α-15β,16β-bisméthylène-19-nor-androst-4-én-3-one,
• 17β-cyano-7β-cyclopropyl-15β,16β-méthylène-19-nor-androst-4-én-3-one,
• 17β-cyano-7α-cyclopropyl-15β,16β-méthylène-19-nor-androst-4-én-3-one,
• 17β-cyano-7β-éthyl-15β,16β-méthylène-19-nor-androst-4-én-3-one,
• 17β-cyano-7α-éthyl-15β,16β-méthylène-19-nor-androst-4-én-3-one,
• 17β-cyano-7β-méthyl-15β,16β-méthylène-19-nor-androst-4-én-3-one,
• 17β-cyano-7α-méthyl-15β,16β-méthylène-19-nor-androst-4-én-3-one,
• 17β-cyano-17α-méthyl-15β,16β-méthylène-19-nor-androsta-4,6-dién-3-one,
• 17β-cyano-17α-éthyl-15β,16β-méthylène-19-nor-androsta-4,6-dién-3-one,
• 17β-cyano-15β,16β-méthylène-7β-vinyl-19-nor-androst-4-én-3-one,
• 17β-cyano-15β,16β-méthylène-7α-vinyl-19-nor-androst-4-én-3-one,
• 17β-cyano-6,6-éthanediyl-15β,16β-méthylène-19-nor-androst-4-én-3-one,
• 17β-cyano-15α,16α-méthylène-19-nor-androst-4-én-3-one,
• 17β-cyano-17α,7α-diméthyl-15β,16β-méthylène-19-nor-androst-4-én-3-one,
• 17β-cyano-17α,7β-diméthyl-15β,16β-méthylène-19-nor-androst-4-én-3-one,
• 17β-cyano-17α-méthyl-7α-éthyl-15β,16β-méthylène-19-nor-androst-4-én-3-one,
• 17β-cyano-17α-méthyl-7β-éthyl-15β,16β-méthylène-19-nor-androst-4-én-3-one,
• 17β-cyano-17α-méthyl-7α-vinyl-15β,16β-méthylène-19-nor-androst-4-én-3-one,
• 17β-cyano-17α-méthyl-7β-vinyl-15β,16β-méthylène-19-nor-androst-4-én-3-one,
• 17β-cyano-17α-méthyl-7α-cyclopropyl-15β,16β-méthylène-19-nor-androst-4-én-3-one,
• 17β-cyano-17α-méthyl-7β-cyclopropyl-15β,16β-méthylène-19-nor-androst-4-én-3-one,
• 17β-cyano-17α-méthyl-6β-hydroxyméthyl-15β,16β-méthylène-19-nor-androst-4-én-3-one,
• 17β-cyano-17α-méthyl-6,6-éthylène-15β,16β-méthylène-19-nor-androst-4-én-3-one,
• 17β-cyano-17α-méthyl-6β,7β-méthylène-15β,16β-méthylène-19-norandrost-4-én-3-one,
• 17β-cyano-17α-méthyl-6α,7α-méthylène-15β,16β-méthylène-19-nor-androst-4-én-3-one,
• 17β-cyano-17α-éthyl-7α-méthyl-15β,16β-méthylène-19-nor-androst-4-én-3-one,
• 17β-cyano-17α-éthyl-7β-méthyl-15β,16β-méthylène-19-nor-androst-4-én-3-one,
• 17β-cyano-17α,7α-diéthyl-15β,16β-méthylène-19-nor-androst-4-én-3-one
• 17β-cyano-17α,7α-diéthyl-15β,16β-méthylène-19-nor-androst-4-én-3-one
• 17β-cyano-17α-éthyl-7α-vinyl-15β,16β-méthylène-19-nor-androst-4-én-3-one,
• 17β-cyano-17α-éthyl-7β-vinyl-15β,16β-méthylène-19-nor-androst-4-én-3-one,
• 17β-cyano-17α-éthyl-7α-cyclopropyl-15β,16β-méthylène-19-nor-androst-4-én-3-one,
• 17β-cyano-17α-éthyl-7β-cyclopropyl-15β,16β-méthylène-19-nor-androst-4-én-3-one,
• 17β-cyano-17α-éthyl-6,6-éthylène-15β,16β-méthylène-19-nor-androst-4-én-3-one,
• 17β-cyano-17α-éthyl-6β,7β-méthylène-15β,16β-méthylène-19-norandrost-4-én-3-one ou
• 17β-cyano-17α-éthyl-6α,7α-méthylène-15β,16β-méthylène-19-nor-androst-4-én-3-one.

2. Utilisation du dérivé de 17β-cyano-19-nor-androst-4-ène selon la revendication 1, pour la fabrication d'un médicament destiné à la contraception orale et au traitement de troubles pré-, péri- et post-ménopausiques.

3. Utilisation selon la revendication 2, **caractérisée en ce que** le médicament a une action progestative et antiminéralocorticoïde.

4. Médicament contenant au moins un dérivé de 17p-cyano-19-nor-androst-4-ène selon la revendication 1 ainsi qu'au moins un additif approprié, pharmaceutiquement acceptable.

5. Médicament selon la revendication 4, contenant en outre au moins un estrogène.

6. Médicament selon la revendication 5, **caractérisé en ce que** l'oestrogène est l'éthinylestradiol.

7. Médicament selon la revendication 5, **caractérisé en ce que** l'oestrogène est un oestrogène naturel.

8. Médicament selon la revendication 7, **caractérisé en ce que** l'oestrogène naturel est l'estradiol.

9. Médicament selon la revendication 7, **caractérisé en ce que** l'oestrogène naturel est le valérate d'estradiol.

10. Médicament selon la revendication 7, **caractérisé en ce que** l'oestrogène naturel est un oestrogène conjugué.
